# EUROPEAN PATENT APPLICATION

(11) **EP 3 792 361 A1**
(43) Date of publication of application: **17.03.2021**
(21) Application number: 19799640.8
(22) Date of filing: 10.05.2019
(51) Int. Cl.: C12N 15/62, A61K 31/7088, A61P 37/08, C12N 1/15, C12N 1/19, C12N 1/21, C12N 5/10, C12N 15/63

(54) **NUCLEIC ACID FOR TREATING CRUSTACEAN ALLERGY**

(30) Priority: 11.05.2018 JP 2018091989
(71) Applicant: Astellas Pharma Inc., Chuo-ku Tokyo 103-8411 (JP)
(72) Inventor: MARUI, Takanori, Tokyo 103-8411 (JP)
(74) Representative: Hoffmann Eitle
(86) International application number: PCT/JP2019/018659
(87) International publication number: WO 2019/216396

(57) **Abstract**

[Problem] To provide a nucleic acid expected to be useful in treating crustacean allergy. [Solution] Provided is a nucleic acid including, in the following order: a base sequence that encodes a signal peptide; a base sequence that encodes a LAMP intra-organelle stabilizing domain; a base sequence that encodes an allergen domain including Litv1, Litv4, and Litv3; a base sequence that encodes a transmembrane domain; and a base sequence that encodes a LAMP endosomal/lysosomal targeting domain, wherein the nucleic acid includes a base sequence that encodes a chimeric protein.

SEQUENCE LISTING

<110>  Astellas Pharma Inc.

<120>  Nucleic acid for treatment of crustacean allergy

<130>  A19003A00

<150>  JP2018-091989
<151>  2018-05-11

<160>  3

<170>  PatentIn version 3.5

<210>  1
<211>  3249
<212>  DNA
<213>  Artificial Sequence

<220>
<223>  Nucleic acid encoding a chimeric protein


<220>
<221>  CDS
<222>  (1)..(3249)

<400>  1
atg gcg ccc cgc agc gcc cgg cga ccc ctg ctg ctg cta ctg ctg ttg      48
Met Ala Pro Arg Ser Ala Arg Arg Pro Leu Leu Leu Leu Leu Leu Leu
1               5                   10                  15

ctg ctg ctc ggc ctc atg cat tgt gcg tca gca gca atg ttt atg gtg      96
Leu Leu Leu Gly Leu Met His Cys Ala Ser Ala Ala Met Phe Met Val
            20                  25                  30

aaa aat ggc aac ggg acc gcg tgc ata atg gcc aac ttc tct gct gcc      144
Lys Asn Gly Asn Gly Thr Ala Cys Ile Met Ala Asn Phe Ser Ala Ala
        35                  40                  45

ttc tca gtg aac tac gac acc aag agt ggc cct aag aac atg acc ctt      192
Phe Ser Val Asn Tyr Asp Thr Lys Ser Gly Pro Lys Asn Met Thr Leu
    50                  55                  60

gac ctg cca tca gat gcc aca gtg gtg ctc aac cgc agc tcc tgt gga      240
Asp Leu Pro Ser Asp Ala Thr Val Val Leu Asn Arg Ser Ser Cys Gly
65                  70                  75                  80

aaa gag aac act tct gac ccc agt ctc gtg att gct ttt gga aga gga      288
Lys Glu Asn Thr Ser Asp Pro Ser Leu Val Ile Ala Phe Gly Arg Gly
                85                  90                  95

cat aca ctc act ctc aat ttc acg aga aat gca aca cgt tac agc gtc      336
His Thr Leu Thr Leu Asn Phe Thr Arg Asn Ala Thr Arg Tyr Ser Val
            100                 105                 110

cag ctc atg agt ttt gtt tat aac ttg tca gac aca cac ctt ttc ccc      384
Gln Leu Met Ser Phe Val Tyr Asn Leu Ser Asp Thr His Leu Phe Pro
        115                 120                 125

aat gcg agc tcc aaa gaa atc aag act gtg gaa tct ata act gac atc      432
Asn Ala Ser Ser Lys Glu Ile Lys Thr Val Glu Ser Ile Thr Asp Ile

130                 135                 140

agg gca gat ata gat aaa aaa tac aga tgt gtt agt ggc acc cag gtc        480
Arg Ala Asp Ile Asp Lys Lys Tyr Arg Cys Val Ser Gly Thr Gln Val
145                 150                 155                 160

cac atg aac aac gtg acc gta acg ctc cat gat gcc acc atc cag gcg        528
His Met Asn Asn Val Thr Val Thr Leu His Asp Ala Thr Ile Gln Ala
                165                 170                 175

tac ctt tcc aac agc agc ttc agc cgg gga gag aca cgc tgt gaa caa        576
Tyr Leu Ser Asn Ser Ser Phe Ser Arg Gly Glu Thr Arg Cys Glu Gln
            180                 185                 190

gac agg cct tcc cca acc aca gcg ccc cct gcg cca ccc agc ccc tcg        624
Asp Arg Pro Ser Pro Thr Thr Ala Pro Pro Ala Pro Pro Ser Pro Ser
        195                 200                 205

ccc tca ccc gtg ccc aag agc ccc tct gtg gac aag tac aac gtg agc        672
Pro Ser Pro Val Pro Lys Ser Pro Ser Val Asp Lys Tyr Asn Val Ser
    210                 215                 220

ggc acc aac ggg acc tgc ctg ctg gcc agc atg ggg ctg cag ctg aac        720
Gly Thr Asn Gly Thr Cys Leu Leu Ala Ser Met Gly Leu Gln Leu Asn
225                 230                 235                 240

ctc acc tat gag agg aag gac aac acg acg gtg aca agg ctt ctc aac        768
Leu Thr Tyr Glu Arg Lys Asp Asn Thr Thr Val Thr Arg Leu Leu Asn
                245                 250                 255

atc aac ccc aac aag acc tcg gcc agc ggg agc tgc ggc gcc cac ctg        816
Ile Asn Pro Asn Lys Thr Ser Ala Ser Gly Ser Cys Gly Ala His Leu
            260                 265                 270

gtg act ctg gag ctg cac agc gag ggc acc acc gtc ctg ctc ttc cag        864
Val Thr Leu Glu Leu His Ser Glu Gly Thr Thr Val Leu Leu Phe Gln
        275                 280                 285

ttc ggg atg aat gca agt tct agc cgg ttt ttc cta caa gga atc cag        912
Phe Gly Met Asn Ala Ser Ser Ser Arg Phe Phe Leu Gln Gly Ile Gln
    290                 295                 300

ttg aat aca att ctt cct gac gcc aga gac cct gcc ttt aaa gct gcc        960
Leu Asn Thr Ile Leu Pro Asp Ala Arg Asp Pro Ala Phe Lys Ala Ala
305                 310                 315                 320

aac ggc tcc ctg cga gcg ctg cag gcc aca gtc ggc aat tcc tac aag       1008
Asn Gly Ser Leu Arg Ala Leu Gln Ala Thr Val Gly Asn Ser Tyr Lys
                325                 330                 335

tgc aac gcg gag gag cac gtc cgt gtc acg aag gcg ttt tca gtc aat       1056
Cys Asn Ala Glu Glu His Val Arg Val Thr Lys Ala Phe Ser Val Asn
            340                 345                 350

ata ttc aaa gtg tgg gtc cag gct ttc aag gtg gaa ggt ggc cag ttt       1104
Ile Phe Lys Val Trp Val Gln Ala Phe Lys Val Glu Gly Gly Gln Phe
        355                 360                 365

ggc tct gtg gag gag tgt ctg ctg gac gag aac agc ctc gag atg gac       1152
Gly Ser Val Glu Glu Cys Leu Leu Asp Glu Asn Ser Leu Glu Met Asp
    370                 375                 380

gcc ata aag aag aaa atg caa gcg atg aaa ctg gag aag gat aac gcc       1200

Ala Ile Lys Lys Lys Met Gln Ala Met Lys Leu Glu Lys Asp Asn Ala
385                 390                 395                 400

atg gat cgg gct gat acg ctg gaa cag cag aat aag gaa gca aac aat      1248
Met Asp Arg Ala Asp Thr Leu Glu Gln Gln Asn Lys Glu Ala Asn Asn
                405                 410                 415

cgt gca gaa aag tcc gaa gaa gag gtt cac aac ctt cag aag aga atg      1296
Arg Ala Glu Lys Ser Glu Glu Glu Val His Asn Leu Gln Lys Arg Met
            420                 425                 430

cag caa ctg gag aat gac ctg gat cag gtg caa gag agc ctg ctg aag      1344
Gln Gln Leu Glu Asn Asp Leu Asp Gln Val Gln Glu Ser Leu Leu Lys
        435                 440                 445

gcc aac atc caa ctg gtc gag aag gat aag gcc ctt agt aac gcc gaa      1392
Ala Asn Ile Gln Leu Val Glu Lys Asp Lys Ala Leu Ser Asn Ala Glu
    450                 455                 460

ggc gag gta gcc gcc ttg aat cgt cgc att caa ctt ctt gag gag gac      1440
Gly Glu Val Ala Ala Leu Asn Arg Arg Ile Gln Leu Leu Glu Glu Asp
465                 470                 475                 480

ctt gag aga tcc gaa gag aga ctg aac acc gct acc acc aaa ctg gca      1488
Leu Glu Arg Ser Glu Glu Arg Leu Asn Thr Ala Thr Thr Lys Leu Ala
                485                 490                 495

gaa gca agc caa gct gcc gat gag tct gaa cgc atg cgc aag gta ttg      1536
Glu Ala Ser Gln Ala Ala Asp Glu Ser Glu Arg Met Arg Lys Val Leu
            500                 505                 510

gag aac agg agc ttg tct gat gag gaa cgc atg gat gcg ttg gag aat      1584
Glu Asn Arg Ser Leu Ser Asp Glu Glu Arg Met Asp Ala Leu Glu Asn
        515                 520                 525

cag ctc aag gaa gca cgg ttc ctg gcc gag gaa gcg gat agg aag tat      1632
Gln Leu Lys Glu Ala Arg Phe Leu Ala Glu Glu Ala Asp Arg Lys Tyr
    530                 535                 540

gat gag gtg gca cga aaa ctc gca atg gtg gag gca gac ttg gaa aga      1680
Asp Glu Val Ala Arg Lys Leu Ala Met Val Glu Ala Asp Leu Glu Arg
545                 550                 555                 560

gct gaa gag aga gct gag aca ggc gag tct aaa atc gtg gaa ctg gag      1728
Ala Glu Glu Arg Ala Glu Thr Gly Glu Ser Lys Ile Val Glu Leu Glu
                565                 570                 575

gaa gaa ctg cgg gtt gtg ggc aac aac ctc aag tca ctg gaa gtg tca      1776
Glu Glu Leu Arg Val Val Gly Asn Asn Leu Lys Ser Leu Glu Val Ser
            580                 585                 590

gaa gag aaa gcc aac caa agg gaa gag gcc tat aaa gag cag atc aaa      1824
Glu Glu Lys Ala Asn Gln Arg Glu Glu Ala Tyr Lys Glu Gln Ile Lys
        595                 600                 605

acc ctg act aac aag ctc aaa gcc gca gag gct cgc gct gaa ttt gct      1872
Thr Leu Thr Asn Lys Leu Lys Ala Ala Glu Ala Arg Ala Glu Phe Ala
    610                 615                 620

gag cgg tca gtg cag aaa ctg cag aag gaa gtg gac agg ctg gag gat      1920
Glu Arg Ser Val Gln Lys Leu Gln Lys Glu Val Asp Arg Leu Glu Asp
625                 630                 635                 640

gag ctc gtc aat gag aaa gag aaa tac aag tcc att aca gac gag ctg 1968
Glu Leu Val Asn Glu Lys Glu Lys Tyr Lys Ser Ile Thr Asp Glu Leu
                645                 650                 655

gat cag acc ttc agt gag ctc tca ggc tac gga gga ggt ggg atg gcc 2016
Asp Gln Thr Phe Ser Glu Leu Ser Gly Tyr Gly Gly Gly Gly Met Ala
            660                 665                 670

tat agc tgg gat aat cgc gtg aag tac gtg gtg cgg tac atg tac gat 2064
Tyr Ser Trp Asp Asn Arg Val Lys Tyr Val Val Arg Tyr Met Tyr Asp
        675                 680                 685

atc gac aac aac ggg ttt ctc gac aag aac gat ttc gaa tgc ctt gcc 2112
Ile Asp Asn Asn Gly Phe Leu Asp Lys Asn Asp Phe Glu Cys Leu Ala
    690                 695                 700

gta cgg aac aca ctg att gag gga agg ggt gag ttt tcc gcc gat gcc 2160
Val Arg Asn Thr Leu Ile Glu Gly Arg Gly Glu Phe Ser Ala Asp Ala
705                 710                 715                 720

tac gcg aac aat cag aaa atc atg cga aac ttg tgg aat gag att gcc 2208
Tyr Ala Asn Asn Gln Lys Ile Met Arg Asn Leu Trp Asn Glu Ile Ala
                725                 730                 735

gaa ctg gcc gac ttc aat aag gat ggg gaa gtt act gtc gac gag ttc 2256
Glu Leu Ala Asp Phe Asn Lys Asp Gly Glu Val Thr Val Asp Glu Phe
            740                 745                 750

aaa cag gca gtg cag aaa cac tgt cag ggc aag aag tat gga gac ttc 2304
Lys Gln Ala Val Gln Lys His Cys Gln Gly Lys Lys Tyr Gly Asp Phe
        755                 760                 765

cca ggc gct ttc aaa gtt ttc ata gcc aac cag ttc aaa gca ata gac 2352
Pro Gly Ala Phe Lys Val Phe Ile Ala Asn Gln Phe Lys Ala Ile Asp
    770                 775                 780

gta aat ggg gat ggg aag gtt ggg ctc gat gag tat cga ctg gac tgc 2400
Val Asn Gly Asp Gly Lys Val Gly Leu Asp Glu Tyr Arg Leu Asp Cys
785                 790                 795                 800

ata act cga tcc gcc ttt gcc gag gtc aaa gag atc gat gac gcg tat 2448
Ile Thr Arg Ser Ala Phe Ala Glu Val Lys Glu Ile Asp Asp Ala Tyr
                805                 810                 815

aac aaa ctg acc act gaa gat gac cgc aag gct ggt ggg ctg aca ctc 2496
Asn Lys Leu Thr Thr Glu Asp Asp Arg Lys Ala Gly Gly Leu Thr Leu
            820                 825                 830

gaa agg tac cag gat ctg tat gcc cag ttt atc agc aat ccc gac gaa 2544
Glu Arg Tyr Gln Asp Leu Tyr Ala Gln Phe Ile Ser Asn Pro Asp Glu
        835                 840                 845

agc tgt tct gcc tgt tac ctc ttt ggc ccg ctg aaa gtc gtc caa gga 2592
Ser Cys Ser Ala Cys Tyr Leu Phe Gly Pro Leu Lys Val Val Gln Gly
    850                 855                 860

gga ggc ggg atg tca cgg aag agc ggc tct cgg agt tcc agc aag agg 2640
Gly Gly Gly Met Ser Arg Lys Ser Gly Ser Arg Ser Ser Ser Lys Arg
865                 870                 875                 880

agc aag aag tct gga ggc ggt tcc aat gtc ttt gac atg ttt acc cag 2688
Ser Lys Lys Ser Gly Gly Gly Ser Asn Val Phe Asp Met Phe Thr Gln
                885                 890                 895

aga cag gtg gct gaa ttt aaa gag gga ttt cag ctg atg gac cgt gac 2736
Arg Gln Val Ala Glu Phe Lys Glu Gly Phe Gln Leu Met Asp Arg Asp
            900                 905                 910

aaa gac gga gtt atc ggt aag act gac ttg aga ggt acg ttc gat gag 2784
Lys Asp Gly Val Ile Gly Lys Thr Asp Leu Arg Gly Thr Phe Asp Glu
        915                 920                 925

att ggc aga atc gcc act gac cag gag ctg gat gag atg ctg gcg gac 2832
Ile Gly Arg Ile Ala Thr Asp Gln Glu Leu Asp Glu Met Leu Ala Asp
    930                 935                 940

gct cct gca ccc atc aat ttc acc atg ctt ctc aat atg ttc gcc gag 2880
Ala Pro Ala Pro Ile Asn Phe Thr Met Leu Leu Asn Met Phe Ala Glu
945                 950                 955                 960

aga caa aca gga gaa agt gac gac gac gac gtg gtg gct aag gct ttc 2928
Arg Gln Thr Gly Glu Ser Asp Asp Asp Asp Val Val Ala Lys Ala Phe
                965                 970                 975

ctg gcc ttt gct gat gag gaa ggg aat att gat tgc gac acg ttc agg 2976
Leu Ala Phe Ala Asp Glu Glu Gly Asn Ile Asp Cys Asp Thr Phe Arg
            980                 985                 990

cat gct ctg atg aca tgg ggc gac aaa ttt agc agt cag gaa gca gac 3024
His Ala Leu Met Thr Trp Gly Asp Lys Phe Ser Ser Gln Glu Ala Asp
        995                 1000                1005

gat gcc ctg gac cag atg gat att gac gac ggc ggg aag atc gat 3069
Asp Ala Leu Asp Gln Met Asp Ile Asp Asp Gly Gly Lys Ile Asp
    1010                1015                1020

gtg cag gga gtc att cag atg ctg aca gca ggc ggt ggg gat gac 3114
Val Gln Gly Val Ile Gln Met Leu Thr Ala Gly Gly Gly Asp Asp
    1025                1030                1035

gca gct gct gaa gag gcg gaa ttc acg ctg atc ccc atc gct gtg 3159
Ala Ala Ala Glu Glu Ala Glu Phe Thr Leu Ile Pro Ile Ala Val
    1040                1045                1050

ggt ggt gcc ctg gcg ggg ctg gtc ctc atc gtc ctc atc gcc tac 3204
Gly Gly Ala Leu Ala Gly Leu Val Leu Ile Val Leu Ile Ala Tyr
    1055                1060                1065

ctc gtc ggc agg aag agg agt cac gca ggc tac cag act atc tag 3249
Leu Val Gly Arg Lys Arg Ser His Ala Gly Tyr Gln Thr Ile
    1070                1075                1080

<210> 2
<211> 1082
<212> PRT
<213> Artificial Sequence

<220>
<223> Synthetic Construct

<400> 2

Met Ala Pro Arg Ser Ala Arg Arg Pro Leu Leu Leu Leu Leu Leu Leu
1               5                   10                  15

Leu Leu Leu Gly Leu Met His Cys Ala Ser Ala Ala Met Phe Met Val
            20                  25                  30

Lys Asn Gly Asn Gly Thr Ala Cys Ile Met Ala Asn Phe Ser Ala Ala
        35                  40                  45

Phe Ser Val Asn Tyr Asp Thr Lys Ser Gly Pro Lys Asn Met Thr Leu
    50                  55                  60

Asp Leu Pro Ser Asp Ala Thr Val Val Leu Asn Arg Ser Ser Cys Gly
65                  70                  75                  80

Lys Glu Asn Thr Ser Asp Pro Ser Leu Val Ile Ala Phe Gly Arg Gly
                85                  90                  95

His Thr Leu Thr Leu Asn Phe Thr Arg Asn Ala Thr Arg Tyr Ser Val
            100                 105                 110

Gln Leu Met Ser Phe Val Tyr Asn Leu Ser Asp Thr His Leu Phe Pro
        115                 120                 125

Asn Ala Ser Ser Lys Glu Ile Lys Thr Val Glu Ser Ile Thr Asp Ile
    130                 135                 140

Arg Ala Asp Ile Asp Lys Lys Tyr Arg Cys Val Ser Gly Thr Gln Val
145                 150                 155                 160

His Met Asn Asn Val Thr Val Thr Leu His Asp Ala Thr Ile Gln Ala
                165                 170                 175

Tyr Leu Ser Asn Ser Ser Phe Ser Arg Gly Glu Thr Arg Cys Glu Gln
            180                 185                 190

Asp Arg Pro Ser Pro Thr Thr Ala Pro Pro Ala Pro Pro Ser Pro Ser
        195                 200                 205

Pro Ser Pro Val Pro Lys Ser Pro Ser Val Asp Lys Tyr Asn Val Ser
    210                 215                 220

Gly Thr Asn Gly Thr Cys Leu Leu Ala Ser Met Gly Leu Gln Leu Asn
225                 230                 235                 240

Leu Thr Tyr Glu Arg Lys Asp Asn Thr Thr Val Thr Arg Leu Leu Asn
                245                 250                 255

Ile Asn Pro Asn Lys Thr Ser Ala Ser Gly Ser Cys Gly Ala His Leu
            260                 265                 270

Val Thr Leu Glu Leu His Ser Glu Gly Thr Thr Val Leu Leu Phe Gln
        275                 280                 285

Phe Gly Met Asn Ala Ser Ser Ser Arg Phe Phe Leu Gln Gly Ile Gln
    290                 295                 300

Leu Asn Thr Ile Leu Pro Asp Ala Arg Asp Pro Ala Phe Lys Ala Ala
305                 310                 315                 320

Asn Gly Ser Leu Arg Ala Leu Gln Ala Thr Val Gly Asn Ser Tyr Lys
                325                 330                 335

Cys Asn Ala Glu Glu His Val Arg Val Thr Lys Ala Phe Ser Val Asn
            340                 345                 350

Ile Phe Lys Val Trp Val Gln Ala Phe Lys Val Glu Gly Gly Gln Phe
        355                 360                 365

Gly Ser Val Glu Glu Cys Leu Leu Asp Glu Asn Ser Leu Glu Met Asp
    370                 375                 380

Ala Ile Lys Lys Lys Met Gln Ala Met Lys Leu Glu Lys Asp Asn Ala
385                 390                 395                 400

Met Asp Arg Ala Asp Thr Leu Glu Gln Gln Asn Lys Glu Ala Asn Asn
                405                 410                 415

Arg Ala Glu Lys Ser Glu Glu Glu Val His Asn Leu Gln Lys Arg Met
            420                 425                 430

Gln Gln Leu Glu Asn Asp Leu Asp Gln Val Gln Glu Ser Leu Leu Lys
        435                 440                 445

Ala Asn Ile Gln Leu Val Glu Lys Asp Lys Ala Leu Ser Asn Ala Glu
    450                 455                 460

Gly Glu Val Ala Ala Leu Asn Arg Arg Ile Gln Leu Leu Glu Glu Asp
465                 470                 475                 480

Leu Glu Arg Ser Glu Glu Arg Leu Asn Thr Ala Thr Thr Lys Leu Ala
                485                 490                 495

Glu Ala Ser Gln Ala Ala Asp Glu Ser Glu Arg Met Arg Lys Val Leu
            500                 505                 510

Glu Asn Arg Ser Leu Ser Asp Glu Glu Arg Met Asp Ala Leu Glu Asn

515                 520                 525

Gln Leu Lys Glu Ala Arg Phe Leu Ala Glu Glu Ala Asp Arg Lys Tyr
    530                 535                 540

Asp Glu Val Ala Arg Lys Leu Ala Met Val Glu Ala Asp Leu Glu Arg
545                 550                 555                 560

Ala Glu Glu Arg Ala Glu Thr Gly Glu Ser Lys Ile Val Glu Leu Glu
                565                 570                 575

Glu Glu Leu Arg Val Val Gly Asn Asn Leu Lys Ser Leu Glu Val Ser
            580                 585                 590

Glu Glu Lys Ala Asn Gln Arg Glu Glu Ala Tyr Lys Glu Gln Ile Lys
        595                 600                 605

Thr Leu Thr Asn Lys Leu Lys Ala Ala Glu Ala Arg Ala Glu Phe Ala
    610                 615                 620

Glu Arg Ser Val Gln Lys Leu Gln Lys Glu Val Asp Arg Leu Glu Asp
625                 630                 635                 640

Glu Leu Val Asn Glu Lys Glu Lys Tyr Lys Ser Ile Thr Asp Glu Leu
                645                 650                 655

Asp Gln Thr Phe Ser Glu Leu Ser Gly Tyr Gly Gly Gly Gly Met Ala
            660                 665                 670

Tyr Ser Trp Asp Asn Arg Val Lys Tyr Val Val Arg Tyr Met Tyr Asp
        675                 680                 685

Ile Asp Asn Asn Gly Phe Leu Asp Lys Asn Asp Phe Glu Cys Leu Ala
    690                 695                 700

Val Arg Asn Thr Leu Ile Glu Gly Arg Gly Glu Phe Ser Ala Asp Ala
705                 710                 715                 720

Tyr Ala Asn Asn Gln Lys Ile Met Arg Asn Leu Trp Asn Glu Ile Ala
                725                 730                 735

Glu Leu Ala Asp Phe Asn Lys Asp Gly Glu Val Thr Val Asp Glu Phe
            740                 745                 750

Lys Gln Ala Val Gln Lys His Cys Gln Gly Lys Lys Tyr Gly Asp Phe
        755                 760                 765

Pro Gly Ala Phe Lys Val Phe Ile Ala Asn Gln Phe Lys Ala Ile Asp
    770                 775                 780

Val Asn Gly Asp Gly Lys Val Gly Leu Asp Glu Tyr Arg Leu Asp Cys
785                 790                 795                 800

Ile Thr Arg Ser Ala Phe Ala Glu Val Lys Glu Ile Asp Asp Ala Tyr
                805                 810                 815

Asn Lys Leu Thr Thr Glu Asp Asp Arg Lys Ala Gly Gly Leu Thr Leu
            820                 825                 830

Glu Arg Tyr Gln Asp Leu Tyr Ala Gln Phe Ile Ser Asn Pro Asp Glu
        835                 840                 845

Ser Cys Ser Ala Cys Tyr Leu Phe Gly Pro Leu Lys Val Val Gln Gly
    850                 855                 860

Gly Gly Gly Met Ser Arg Lys Ser Gly Ser Arg Ser Ser Ser Lys Arg
865                 870                 875                 880

Ser Lys Lys Ser Gly Gly Gly Ser Asn Val Phe Asp Met Phe Thr Gln
                885                 890                 895

Arg Gln Val Ala Glu Phe Lys Glu Gly Phe Gln Leu Met Asp Arg Asp
            900                 905                 910

Lys Asp Gly Val Ile Gly Lys Thr Asp Leu Arg Gly Thr Phe Asp Glu
        915                 920                 925

Ile Gly Arg Ile Ala Thr Asp Gln Glu Leu Asp Glu Met Leu Ala Asp
    930                 935                 940

Ala Pro Ala Pro Ile Asn Phe Thr Met Leu Leu Asn Met Phe Ala Glu
945                 950                 955                 960

Arg Gln Thr Gly Glu Ser Asp Asp Asp Asp Val Val Ala Lys Ala Phe
                965                 970                 975

Leu Ala Phe Ala Asp Glu Glu Gly Asn Ile Asp Cys Asp Thr Phe Arg
            980                 985                 990

His Ala Leu Met Thr Trp Gly Asp  Lys Phe Ser Ser Gln  Glu Ala Asp
        995                 1000                1005

Asp Ala  Leu Asp Gln Met Asp  Ile Asp Asp Gly Gly  Lys Ile Asp
    1010                1015                1020

Val Gln Gly Val Ile Gln Met Leu Thr Ala Gly Gly Gly Asp Asp
    1025                1030                1035

Ala Ala Ala Glu Glu Ala Glu Phe Thr Leu Ile Pro Ile Ala Val
    1040                1045                1050

Gly Gly Ala Leu Ala Gly Leu Val Leu Ile Val Leu Ile Ala Tyr
    1055                1060                1065

Leu Val Gly Arg Lys Arg Ser His Ala Gly Tyr Gln Thr Ile
    1070                1075                1080

<210> 3
<211> 6253
<212> DNA
<213> Artificial Sequence

<220>
<223> A vector comprising nucleic acid encoding a chimeric protein

<400> 3
ccgcctaatg agcgggcttt tttttcttag ggtgcaaaag gagagcctgt aagcgggcac      60

tcttccgtgg tctggtggat aaattcgcaa gggtatcatg gcggacgacc ggggttcgag     120

ccccgtatcc ggccgtccgc cgtgatccat gcggttaccg cccgcgtgtc gaacccaggt     180

gtgcgacgtc agacaacggg ggagtgctcc ttttggcttc cttccccttc ttccgcttcc     240

tcgctcactg actcgctgcg ctcggtcgtt cggctgcggc gagcggtatc agctcactca     300

aaggcggtaa tacggttatc cacagaatca ggggataacg caggaaagaa catgtgagca     360

aaaggccagc aaaaggccag gaaccgtaaa aaggccgcgt tgctggcgtt tttccatagg     420

ctccgccccc ctgacgagca tcacaaaaat cgacgctcaa gtcagaggtg gcgaaacccg     480

acaggactat aaagatacca ggcgtttccc cctggaagct ccctcgtgcg ctctcctgtt     540

ccgaccctgc cgcttaccgg atacctgtcc gcctttctcc cttcgggaag cgtggcgctt     600

tctcatagct cacgctgtag gtatctcagt tcggtgtagg tcgttcgctc caagctgggc     660

tgtgtgcacg aaccccccgt tcagcccgac cgctgcgcct tatccggtaa ctatcgtctt     720

gagtccaacc cggtaagaca cgacttatcg ccactggcag cagccactgg taacaggatt     780

agcagagcga ggtatgtagg cggtgctaca gagttcttga agtggtggcc taactacggc     840

tacactagaa gaacagtatt tggtatctgc gctctgctga agccagttac cttcggaaaa     900

agagttggta gctcttgatc cggcaaacaa accaccgctg gtagcggtgg tttttttgtt     960

tgcaagcagc agattacgcg cagaaaaaaa ggatctcaag aagatccttt gatcttttct    1020

acggggtctg acgctcagtg gaacgaaaac tcacgttaag ggattttggt catgagatta    1080

tcaaaaagga tcttcaccta gatcctttta aattaaaaat gaagttttaa atcaatctaa    1140

agtatatatg agtaaacttg gtctgacagt taccaatgct taatcagtga ggcacctatc 1200

tcagcgatct gtctatttcg ttcatccata gttgcctgac tcctgcaaac cacgttgtgg 1260

tagaattggt aaagagagtc gtgtaaaata tcgagttcgc acatcttgtt gtctgattat 1320

tgatttttgg cgaaaccatt tgatcatatg acaagatgtg tatctacctt aacttaatga 1380

ttttgataaa aatcattagg taccccggct ctagatggca tgacattaac ctataaaaat 1440

aggcgtatca cgaggccctt tcgtctcgcg cgtttcggtg atgacggtga aaacctctga 1500

cacatgcagc tcccggagac ggtcacagct tgtctgtaag cggatgccgg gagcagacaa 1560

gcccgtcagg gcgcgtcagc gggtgttggc gggtgtcggg gctggcttaa ctatgcggca 1620

tcagagcaga ttgtactgag agtgcaccat atgcggtgtg aaataccgca cagatgcgta 1680

aggagaaaat accgcatcag attggctatt ggccattgca tacgttgtat ccatatcata 1740

atatgtacat ttatattggc tcatgtccaa cattaccgcc atgttgacat tgattattga 1800

ctagttatta atagtaatca attacggggt cattagttca tagcccatat atggagttcc 1860

gcgttacata acttacggta aatggcccgc ctggctgacc gcccaacgac ccccgcccat 1920

tgacgtcaat aatgacgtat gttcccatag taacgccaat agggactttc cattgacgtc 1980

aatgggtgga gtatttacgg taaactgccc acttggcagt acatcaagtg tatcatatgc 2040

caagtacgcc ccctattgac gtcaatgacg gtaaatggcc cgcctggcat tatgcccagt 2100

acatgacctt atgggacttt cctacttggc agtacatcta cgtattagtc atcgctatta 2160

ccatggtgat gcggttttgg cagtacatca atgggcgtgg atagcggttt gactcacggg 2220

gatttccaag tctccacccc attgacgtca atgggagttt gttttggcac caaaatcaac 2280

gggactttcc aaaatgtcgt aacaactccg ccccattgac gcaaatgggc ggtaggcgtg 2340

tacggtggga ggtctatata agcagagctc gtttagtgaa ccgtcagatc gcctggagac 2400

gccatccacg ctgttttgac ctccatagaa gacaccggga ccgatccagc ctccgcggct 2460

cgcatctctc cttcacgcgc ccgccgccct acctgaggcc gccatccacg ccggttgagt 2520

cgcgttctgc cgcctcccgc ctgtggtgcc tcctgaactg cgtccgccgt ctaggtaagt 2580

ttaaagctca ggtcgagacc gggcctttgt ccggcgctcc cttggagcct acctagactc 2640

agccggctct ccacgctttg cctgaccctg cttgctcaac tctagttctc tcgttaactt 2700

aatgagacag atagaaactg gtcttgtaga aacagagtag tcgcctgctt ttctgccagg 2760

tgctgacttc tctcccctgg gcttttttct ttttctcagg ttgaaaagaa gaagacgaag 2820

aagacgaaga agacaaaccg tcgtcgacat ggcgccccgc agcgcccggc gacccctgct 2880

gctgctactg ctgttgctgc tgctcggcct catgcattgt gcgtcagcag caatgtttat 2940

ggtgaaaaat ggcaacggga ccgcgtgcat aatggccaac ttctctgctg ccttctcagt 3000

gaactacgac accaagagtg gccctaagaa catgaccctt gacctgccat cagatgccac 3060

agtggtgctc aaccgcagct cctgtggaaa agagaacact tctgacccca gtctcgtgat 3120

tgcttttgga agaggacata cactcactct caatttcacg agaaatgcaa cacgttacag 3180

cgtccagctc atgagttttg tttataactt gtcagacaca caccttttcc ccaatgcgag 3240

ctccaaagaa atcaagactg tggaatctat aactgacatc agggcagata tagataaaaa 3300

atacagatgt gttagtggca cccaggtcca catgaacaac gtgaccgtaa cgctccatga 3360

tgccaccatc caggcgtacc tttccaacag cagcttcagc cggggagaga cacgctgtga 3420

acaagacagg ccttccccaa ccacagcgcc ccctgcgcca cccagcccct cgccctcacc 3480

cgtgcccaag agcccctctg tggacaagta caacgtgagc ggcaccaacg ggacctgcct 3540

gctggccagc atggggctgc agctgaacct cacctatgag aggaaggaca acacgacggt 3600

gacaaggctt ctcaacatca accccaacaa gacctcggcc agcgggagct gcggcgccca 3660

cctggtgact ctggagctgc acagcgaggg caccaccgtc ctgctcttcc agttcgggat 3720

gaatgcaagt tctagccggt ttttcctaca aggaatccag ttgaatacaa ttcttcctga 3780

cgccagagac cctgccttta aagctgccaa cggctccctg cgagcgctgc aggccacagt 3840

cggcaattcc tacaagtgca acgcggagga gcacgtccgt gtcacgaagg cgttttcagt 3900

caatatattc aaagtgtggg tccaggcttt caaggtggaa ggtggccagt ttggctctgt 3960

ggaggagtgt ctgctggacg agaacagcct cgagatggac gccataaaga agaaaatgca 4020

agcgatgaaa ctggagaagg ataacgccat ggatcgggct gatacgctgg aacagcagaa 4080

taaggaagca aacaatcgtg cagaaaagtc cgaagaagag gttcacaacc ttcagaagag 4140

aatgcagcaa ctggagaatg acctggatca ggtgcaagag agcctgctga aggccaacat 4200

ccaactggtc gagaaggata aggcccttag taacgccgaa ggcgaggtag ccgccttgaa 4260

tcgtcgcatt caacttcttg aggaggacct tgagagatcc gaagagagac tgaacaccgc 4320

taccaccaaa ctggcagaag caagccaagc tgccgatgag tctgaacgca tgcgcaaggt 4380

attggagaac aggagcttgt ctgatgagga acgcatggat gcgttggaga atcagctcaa 4440

ggaagcacgg ttcctggccg aggaagcgga taggaagtat gatgaggtgg cacgaaaact 4500

cgcaatggtg gaggcagact tggaaagagc tgaagagaga gctgagacag gcgagtctaa 4560

aatcgtggaa ctggaggaag aactgcgggt tgtgggcaac aacctcaagt cactggaagt 4620

gtcagaagag aaagccaacc aaagggaaga ggcctataaa gagcagatca aaaccctgac 4680

taacaagctc aaagccgcag aggctcgcgc tgaatttgct gagcggtcag tgcagaaact 4740

gcagaaggaa gtggacaggc tggaggatga gctcgtcaat gagaaagaga aatacaagtc 4800

cattacagac gagctggatc agaccttcag tgagctctca ggctacggag gaggtgggat 4860

ggcctatagc tgggataatc gcgtgaagta cgtggtgcgg tacatgtacg atatcgacaa 4920

caacgggttt ctcgacaaga acgatttcga atgccttgcc gtacggaaca cactgattga 4980

gggaaggggt gagttttccg ccgatgccta cgcgaacaat cagaaaatca tgcgaaactt 5040

gtggaatgag attgccgaac tggccgactt caataaggat ggggaagtta ctgtcgacga 5100

gttcaaacag gcagtgcaga aacactgtca gggcaagaag tatggagact tcccaggcgc 5160

tttcaaagtt ttcatagcca accagttcaa agcaatagac gtaaatgggg atgggaaggt 5220

tgggctcgat gagtatcgac tggactgcat aactcgatcc gcctttgccg aggtcaaaga 5280

gatcgatgac gcgtataaca aactgaccac tgaagatgac cgcaaggctg gtgggctgac 5340

actcgaaagg taccaggatc tgtatgccca gtttatcagc aatcccgacg aaagctgttc 5400

tgcctgttac ctctttggcc cgctgaaagt cgtccaagga ggaggcggga tgtcacggaa 5460

gagcggctct cggagttcca gcaagaggag caagaagtct ggaggcggtt ccaatgtctt 5520

tgacatgttt acccagagac aggtggctga atttaaagag ggatttcagc tgatggaccg 5580

tgacaaagac ggagttatcg gtaagactga cttgagaggt acgttcgatg agattggcag 5640

aatcgccact gaccaggagc tggatgagat gctggcggac gctcctgcac ccatcaattt 5700

caccatgctt ctcaatatgt tcgccgagag acaaacagga gaaagtgacg acgacgacgt 5760

ggtggctaag gctttcctgg cctttgctga tgaggaaggg aatattgatt gcgacacgtt 5820

caggcatgct ctgatgacat ggggcgacaa atttagcagt caggaagcag acgatgccct 5880

ggaccagatg gatattgacg acggcgggaa gatcgatgtg cagggagtca ttcagatgct 5940

gacagcaggc ggtggggatg acgcagctgc tgaagaggcg gaattcacgc tgatccccat 6000

cgctgtgggt ggtgccctgg cggggctggt cctcatcgtc ctcatcgcct acctcgtcgg 6060

caggaagagg agtcacgcag gctaccagac tatctagtaa ggatcttttt ccctctgcca 6120

aaaattatgg ggacatcatg aagccccttg agcatctgac ttctggctaa taaaggaaat 6180

ttattttcat tgcaatagtg tgttggaatt ttttgtgtct ctcactcgga aggacataag 6240

ggcggccgct agc 6253

## Description

### Technical Field

The present invention relates to a nucleic acid which is expected to be useful as an active ingredient of a pharmaceutical composition, for example, a nucleic acid which is expected to be useful for treating crustacean allergy.

### Background Art

Food allergy refers to a "phenomenon that causes adverse symptoms to a organisms through an antigen-specific immunological mechanism caused by food", which is characterized by symptoms such as urticaria, eczema, diarrhea, cough, and the like due to food ingestion. The crustacean allergy is one of the major food allergies in adults, and as allergens that cause the crustacean allergy, a wide variety of allergens including Lit v 1 and Pen m 1 classified into Tropomyosin, Lit v 2 and Pen m 2 classified into Arginine kinase, Lit v 3 and Pen m 3 classified into Myosin light chain, and Lit v 4 and Pen m 4 classified into Sarcoplasmic calcium binding protein, and the like are known (Non-Patent Documents 1 to 4).

Currently, there is no fundamental treatment method for the crustacean allergy, and avoidance from allergen ingestion by removing allergic food is the only way to prevent a crustacean allergy symptom such as anaphylaxis. Although allergen specific immunotherapy (ASIT), DNA plasmids, Peptide-based immunotherapy (PIT), and the like have been studied, clinically effective treatment methods have not been developed yet, and therefore, effective treatment methods have been required (Non-Patent Document 4, European Annals of Allergy and Clinical Immunology, 2017, Vol. 49, p. 252-256).

The allergic disease is caused by the following steps: 1) allergens taken into a body are phagocytosed by antigen-presenting cells and presented to naive T cells, 2) the naive T cells are differentiated into Th2 cells, 3) cytokine such as IL-4 is produced from an immune cell such as the Th2 cell, 4) B cells produce IgE by IL-4, and 5) IgE binding to the allergens binds to mast cells. It has been known that in allergic patients, the balance between Th1-type immunity involving Th1 cells producing IFN-γ or the like and Th2-type immunity involving Th2 cells producing IL-4 or the like shifts to Th2-type dominant, which results in Th2-type inflammatory immune response (Middleton's Allergy Seventh edition Principles & Practice, 2009). Thus, IFN-γ can be used as an indicator of Th1-type immunity, and IL-4 can be used as an indicator of Th2-type immunity. Further, in mice, IFN-γ causes a preferential class switch to IgG2a isotype in activated B cells, while suppresses responses to all the other isotypes. That is, production of IgG2a antibody can also be used as an indicator of Th1-type immunity. For example, it has been known that production of IgG2a antibody is promoted in IL-4-deficient mice and that IgG2a antibody production is suppressed in IFN-γ-deficient mice (Arthritis Res., 2002, Vol. 4, p. 54-58). There is also a report that antibodies produced by B cells are involved in the mechanism of action of allergen immunotherapy. For example, it has been known that in humans, IgG antagonizes IgE binding to an allergen to inhibit formation of allergen-IgE complex and thereby inhibit histamine release from mast cells (Journal of Allergy and Clinical Immunology, 2017, Vol. 140, p. 1485-1498).

As one of the techniques for nucleic acid vaccines, nucleic acid vaccines for treating allergy using lysosome-associated membrane proteins (LAMP) have been studied. Further, a plasmid comprising a nucleic acid encoding a chimeric protein comprising LAMP-1, which is a member of LAMP family, and Cry J1 and/or Cry J2, which are allergens of Cryptomeria japonica, was constructed (Patent Document 1 and Non-Patent Document 5). It has been reported that such a plasmid does not cause systemic release of free allergen which causes anaphylaxis but induces a Th1-type immune response. Furthermore, it has been reported that a plasmid comprising a nucleic acid encoding a chimeric protein comprising LAMP-1 and peanut allergens Ara HI, Ara H2 and Ara H3 reduced production of IgE in a mouse model (Patent Document 2). However, a nucleic acid vaccine for treating crustacean allergy has not been reported yet.

### Related Art

### Patent Document

[Patent Document 1] WO 2013/187906
[Patent Document 2] WO 2015/200357

### Non-Patent Document

[Non-Patent Document 1] "Allergo Journal International", (Germany), 2016; 25(7): 210-218.
[Non-Patent Document 2] "The Journal of Allergy and Clinical Immunology", (USA), 2009; 124(1): 114-120.
[Non-Patent Document 3] "The Journal of Allergy and Clinical Immunology", (USA), 2008; 122(4): 795-802.
[Non-Patent Document 4] "Clinical Reviews in Allergy and Immunology", (USA), 2015; 49(2): 203-216.
[Non-Patent Document 5] "Journal of Immunology Research", (Egypt), 2016; Article ID 4857869

### Disclosure of Invention

### Technical Problem

An object of the present invention is to provide a nucleic acid which is expected to be useful for treating crustacean allergy.

### Means for Solving the Problems

As a result of repeated investigation with considerable creativity in the preparation of nucleic acids for treating crustacean allergy, the present inventors have prepared LAMP-Lit v 1-Lit v 4-Lit v 3 plasmid (Example 1), confirmed that a chimeric protein is expressed from the plasmid (Example 2), and found that a Th1-type immune response is induced in mice to which the plasmid is administered (Examples 3 and 4). As a result, a nucleic acid which is expected to be useful for treating crustacean allergy is provided, and thereby the present invention has been completed. Furthermore, it is found that allergic symptoms caused by shrimp antigen challenge are alleviated in mice administered with the plasmid (Example 5).

That is, the present invention relates to the following [1] to [17].
[1] A nucleic acid comprising:
   a nucleotide sequence encoding a chimeric protein,
   wherein the nucleotide sequence is a nucleotide sequence comprising the following nucleotide sequences in this order:
   a nucleotide sequence encoding a signal peptide;
   a nucleotide sequence encoding an intra-organelle stabilizing domain of LAMP;
   a nucleotide sequence encoding an allergen domain comprising Lit v 1, Lit v 4, and Lit v 3;
   a nucleotide sequence encoding a transmembrane domain; and
   a nucleotide sequence encoding an endosomal/lysosomal targeting domain of LAMP.
[2] A nucleic acid comprising:
   a nucleotide sequence encoding a chimeric protein,
   wherein the nucleotide sequence is a nucleotide sequence comprising the following nucleotide sequences in this order:
      a nucleotide sequence encoding a signal peptide;
      a nucleotide sequence encoding an intra-organelle stabilizing domain of LAMP;
      a nucleotide sequence encoding an allergen domain comprising Lit v 1, Lit v 4, and Lit v 3 in this order;
      a nucleotide sequence encoding a transmembrane domain; and
      a nucleotide sequence encoding an endosomal/lysosomal targeting domain of LAMP.
[3] The nucleic acid described in [1] or [2], wherein the signal peptide is a signal peptide of LAMP.
[4] The nucleic acid described in any one of [1] to [3], wherein the transmembrane domain is a transmembrane domain of LAMP.
[5] The nucleic acid described in any one of [1] to [4], wherein the signal peptide consists of an amino acid sequence of amino acid numbers 1 to 27 of SEQ ID NO: 2, the intra-organelle stabilizing domain consists of an amino acid sequence of amino acid numbers 28 to 380 of SEQ ID NO: 2, the allergen domain is an allergen domain comprising Lit v 1 consisting of an amino acid sequence of amino acid numbers 383 to 666 of SEQ ID NO: 2, Lit v 4 consisting of an amino acid sequence of amino acid numbers 671 to 863 of SEQ ID NO: 2, and Lit v 3 consisting of an amino acid sequence of amino acid numbers 868 to 1044 of SEQ ID NO: 2, the transmembrane domain consists of an amino acid sequence of amino acid numbers 1048 to 1070 of SEQ ID NO: 2, and the endosomal/lysosomal targeting domain consists of an amino acid sequence of amino acid numbers 1079 to 1082 of SEQ ID NO: 2.
[6] A nucleic acid comprising:
   a nucleotide sequence encoding a chimeric protein consisting of an amino acid sequence having at least 90% identity to an amino acid sequence shown by SEQ ID NO: 2, wherein the nucleic acid has an action of inducing Th1-type immunity to an allergen selected from the group consisting of Lit v 1, Lit v 4, and Lit v 3.
[7] A nucleic acid comprising:
   a) a nucleotide sequence encoding a chimeric protein consisting of an amino acid sequence shown by SEQ ID NO: 2; or
   b) a nucleotide sequence encoding a chimeric protein consisting of an amino acid sequence in which 1 to 10 amino acids are deleted, substituted, inserted, and/or added in the amino acid sequence shown by SEQ ID NO: 2, wherein the nucleic acid has an action of inducing Th1-type immunity to an allergen selected from the group consisting of Lit v 1, Lit v 4, and Lit v 3.
[8] A nucleic acid comprising:
   a nucleotide sequence encoding a chimeric protein consisting of an amino acid sequence shown by SEQ ID NO: 2.
[9] An expression vector comprising:
   the nucleic acid described in any one of [1] to [8].
[10] An expression vector comprising:
   the nucleic acid described in [8].
[11] A host cell transformed with the nucleic acid described in any one of [1] to [8].
[12] A method for producing a nucleic acid, comprising:
   culturing a host cell transformed with the nucleic acid described in any one of [1] to [8].
[13] A pharmaceutical composition comprising:
   the expression vector described in [10] and a pharmaceutically acceptable excipient.
[14] The pharmaceutical composition described in [13], which is a pharmaceutical composition for preventing or treating crustacean allergy.
[15] A method for preventing or treating crustacean allergy, comprising:
   administering a prophylactically effective or therapeutically effective amount of the expression vector described in [10].
[16] The expression vector described in [10], for use in preventing or treating crustacean allergy.
[17] Use of the expression vector described in [10] for the manufacture of a pharmaceutical composition for preventing or treating crustacean allergy.

### Effects of the Invention

The nucleic acid of the present invention can be used for preventing or treating crustacean allergy.

### Brief Description of Drawings

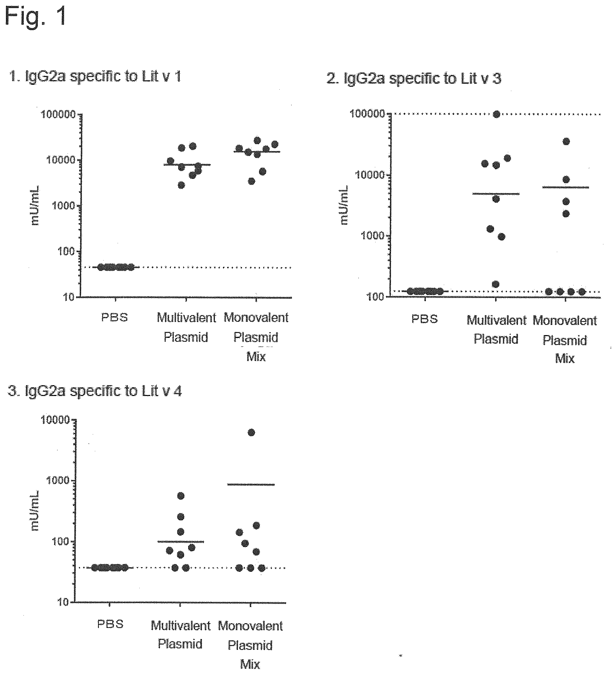

[Fig. 1] Fig. 1 is a diagram illustrating production of IgG2a antibody specific to Lit v 1, Lit v 3, and Lit v 4, which is induced when the nucleic acid of the present invention was administered to a mouse. The vertical axis indicates plasma antibody titer (mU/mL), and the horizontal axis indicates each administration group. The dotted line represents a detection limit, and the horizontal line represents the geometric mean value of each administration group.

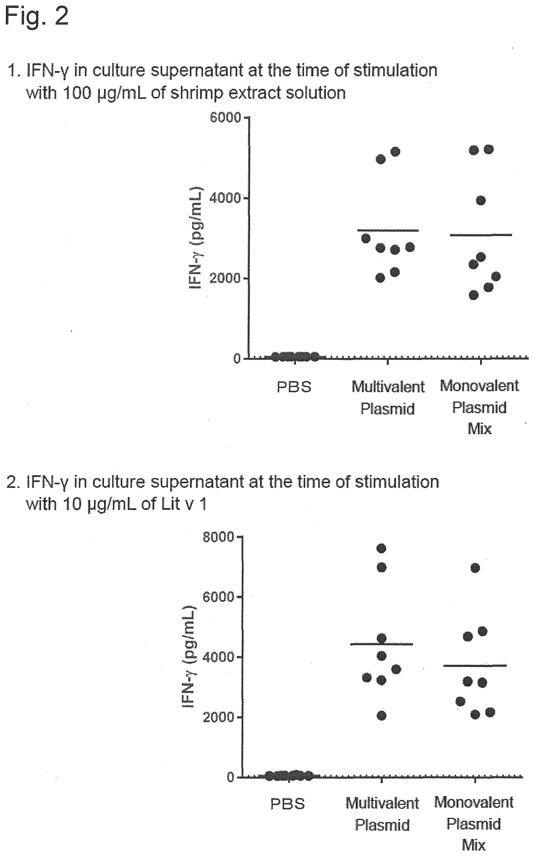

[Fig. 2] Fig. 2 is a diagram illustrating IFN-γ production when splenocytes of the mouse administered with the nucleic acid of the present invention were stimulated with a shrimp extract solution having a final concentration of 100 µg/mL or a Lit v 1 protein having a final concentration of 10 µg/mL. The vertical axis indicates the concentration of IFN-γ in the culture supernatant (pg/mL), and the horizontal axis indicates each administration group. The dotted line shows the detection limit, and the horizontal line shows the arithmetic mean value of each administration group.

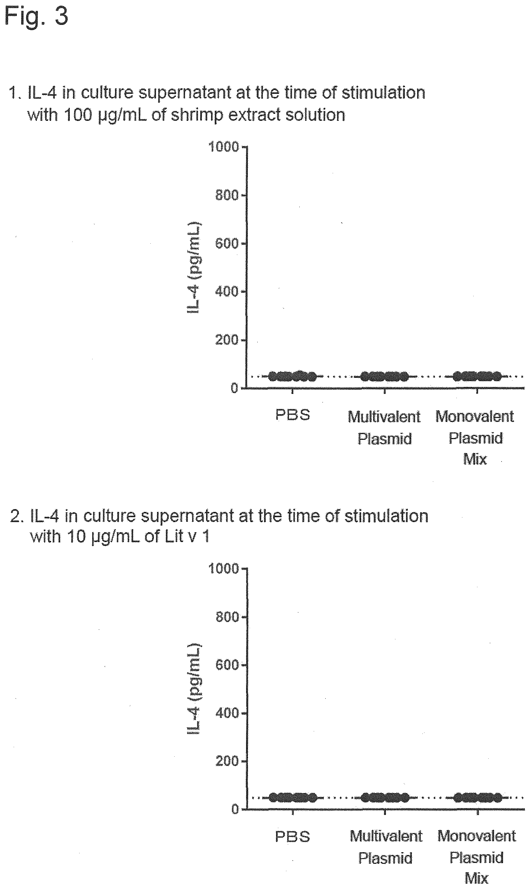

[Fig. 3] Fig. 3 is a diagram illustrating IL-4 production when splenocytes of the mouse administered with the nucleic acid of the present invention were stimulated with a shrimp extract solution having a final concentration of 100 µg/mL or a Lit v 1 protein having a final concentration of 10 µg/mL. The vertical axis indicates the concentration of IL-4 in the culture supernatant (pg/mL), and the horizontal axis indicates each administration group. The dotted line shows the detection limit, and the horizontal line shows the arithmetic mean value of each administration group.

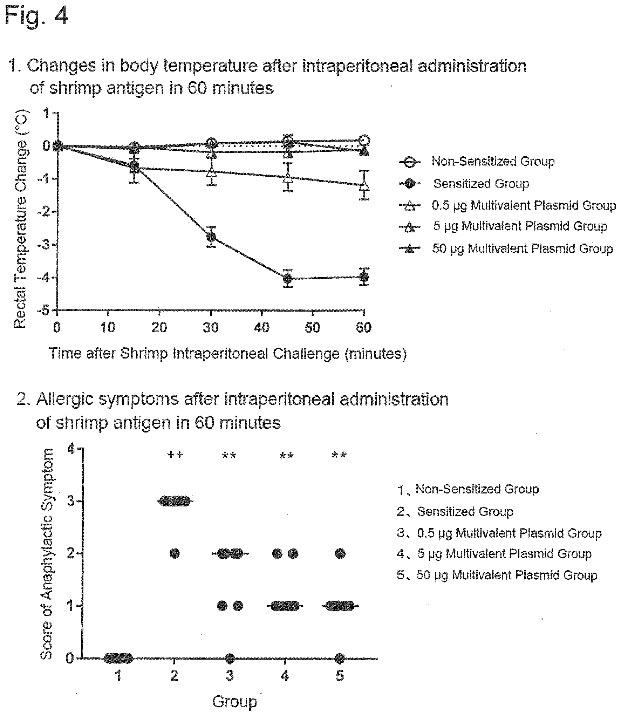

[Fig. 4] Fig. 4 illustrates scores of changes in body temperature and allergic symptoms after intraperitoneal administration of shrimp antigen when the nucleic acid of the present invention is administered to a shrimp antigen-sensitized mouse. The change in body temperature indicates the change from the pre-administration body temperature at the points immediately before intraperitoneal administration of shrimp antigen as well as after 15 minutes, 30 minutes, 45 minutes, and 60 minutes. The vertical axis indicates rectal temperature change (°C), and the horizontal axis indicates time. The plot indicates the value of arithmetic mean within the same prescription at each time point, and the vertical line indicates a standard error. The allergic symptom indicates the score of each mouse in which the symptom observed 60 minutes after the intraperitoneal administration of the shrimp antigen was determined based on criteria of a document (The Journal of Allergy and Clinical Immunology, 2013, Vol. 131, p. 213-221). The vertical axis indicates an anaphylactic symptom score, and the horizontal axis indicates each administration group. The horizontal line indicates a median value of each administration group. ++ indicates that a P value is less than 0.01 in the significant difference test by the Willcoxon rank sum test for a non-sensitized group, and ** indicates that a P value is less than 0.01 in the significant difference test by the Steel multiple comparison test for a sensitized group.

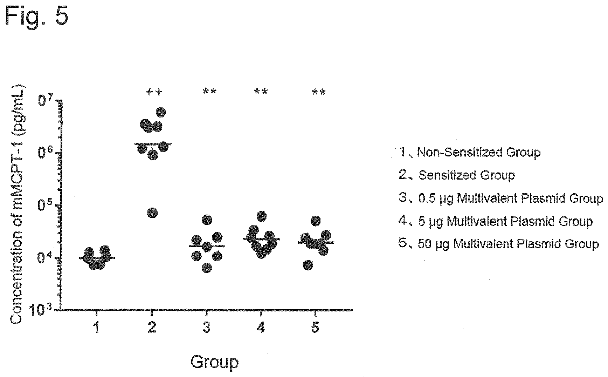

[Fig. 5] Fig. 5 illustrates a concentration of mouse mast cell protease 1 (mMCPT-1) in plasma when the nucleic acid of the present invention was administered to the shrimp antigen-sensitized mouse. The vertical axis indicates the concentration of mMCPT-1 (pg/mL), and the horizontal axis indicates each administration group. The horizontal line indicates a geometric mean value of each administration group, ++ indicates that a P value is less than 0.01 in the significance difference test by the Unpaired t test for the non-sensitized group after logarithmic transformation, and ** indicates that a P value is less than 0.01 in the significant difference test by the Dunnett's multiple comparison test for a sensitized group after logarithmic transformation.

### Embodiments for Carrying Out the Invention

Hereinafter, the present invention will be described in detail.

### <Nucleic acid of the present invention>

Examples of the nucleic acid of the present invention include a nucleic acid having the following features:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide;
a nucleotide sequence encoding an intra-organelle stabilizing domain of LAMP;
a nucleotide sequence encoding an allergen domain comprising Lit v 1, Lit v 4, and Lit v 3;
a nucleotide sequence encoding a transmembrane domain; and
a nucleotide sequence encoding an endosomal/lysosomal targeting domain of LAMP.

In the present invention, the nucleic acid is a polymer which is formed by polymerization of nucleotides and consists of a nucleotide sequence with an arbitrary length. The nucleotides can include deoxyribonucleotides, ribonucleotides, and/or their analogs. The nucleic acid of the present invention is DNA, RNA or modified a nucleic acid thereof. In one embodiment, the nucleic acid of the present invention is DNA.

In one embodiment, the nucleic acid of the present invention is a nucleic acid introduced into an expression vector. In one embodiment, the nucleic acid of the present invention is a nucleic acid introduced into a plasmid vector.

In the specification, "chimeric protein" means a protein encoded by a nucleotide sequence in which two or more genes are fused by using genetic recombination technology. The nucleic acid of the present invention includes a nucleotide sequence encoding chimeric protein comprising a signal peptide, an intra-organelle stabilizing domain of LAMP, an allergen domain comprising Lit v 1, Lit v 4, and Lit v 3, a transmembrane domain, and an endosomal/lysosomal targeting domain of LAMP in this order (hereinafter, referred to as "chimeric protein relating to the present invention").

LAMP is well-known protein to those skilled in the art (J Biol Chem., 1991, Vol. 266, p. 21327-21330). In the present specification, LAMP is not particularly limited, but examples thereof include LAMP-1, LAMP-2, CD63/LAMP-3, DC-LAMP, and LIMP II, and homologs, orthologs, paralogs, variants, and modified proteins thereof. In one embodiment of the present invention, LAMP is LAMP-1. In the present invention, an animal from which LAMP is derived is not particularly limited, but in one embodiment, LAMP is human LAMP. In one embodiment, human LAMP is human LAMP-1. Examples of an amino acid sequence of human LAMP-1 include an amino acid sequence in which the amino acid sequence shown by amino acid numbers 1047 to 1082 of SEQ ID NO: 2 is bound to a C-terminal of the amino acid sequence shown by amino acid numbers 1 to 380 of SEQ ID NO: 2.

The general structure of the signal peptide is well known to those skilled in the art (Annu Rev Biochem., 2003, Vol. 72, p. 395-447). The signal peptide has a function of directing transport and localization of a protein. As the signal peptide used in the present invention, any suitable signal peptide can be selected as long as it has a function of directing transport and localization of the protein. In one embodiment, the signal peptide used in the present invention is a signal peptide of LAMP. In one embodiment, the signal peptide of LAMP used in the present invention is a signal peptide of LAMP-1.

In one embodiment, the signal peptide used in the present invention consists of the following amino acid sequence of (a) or (b):
(a) an amino acid sequence having at least 90% identity to the amino acid sequence of amino acid numbers 1 to 27 of SEQ ID NO: 2; or
(b) the amino acid sequence of amino acid numbers 1 to 27 of SEQ ID NO: 2, or an amino acid sequence in which 1 to 3 amino acids are deleted, substituted, inserted and/or added in the amino acid sequence of amino acid numbers 1 to 27 of SEQ ID NO: 2.

The term of "identity" in the present specification means a value of Identity obtained by using an EMBOSS Needle (Nucleic Acids Res., 2015, Vol.43, p. W580-W584; https://www.ebi.ac.uk/Tools/psa/emboss_needle/) with a parameter prepared by default. The above parameters are as follows.
Gap Open Penalty = 10
Gap Extend Penalty = 0.5
Matrix = EBLOSUM62
End Gap Penalty = false

In one embodiment, the signal peptide used in the present invention consists of the amino acid sequence of amino acid numbers 1 to 27 of SEQ ID NO: 2.

The sequence of the intra-organelle stabilizing domain of LAMP is well known to those skilled in the art (WO 2013/187906). The intra-organelle stabilizing domain of LAMP has a function of protecting the allergen domain from proteases, low pH, and other substances and conditions that destabilize a protein. As the intra-organelle stabilizing domain of LAMP used in the present invention, any suitable intra-organelle stabilizing domain of LAMP can be selected as long as it has a function of protecting the allergen domain from proteases, low pH, and other substances and conditions that destabilize a protein. In one embodiment, the intra-organelle stabilizing domain of LAMP used in the present invention is an intra-organelle stabilizing domain of LAMP-1.

In one embodiment, the intra-organelle stabilizing domain of LAMP used in the present invention consists of the following amino acid sequence of (a) or (b):
(a) an amino acid sequence having at least 90% identity to the amino acid sequence of amino acid numbers 28 to 380 of SEQ ID NO: 2; or
(b) the amino acid sequence of amino acid numbers 28 to 380 of SEQ ID NO: 2, or an amino acid sequence in which 1 to 10 amino acids are deleted, substituted, inserted and/or added in the amino acid sequence of amino acid numbers 28 to 380 of SEQ ID NO: 2.

In one embodiment, the intra-organelle stabilizing domain of LAMP used in the present invention consists of the amino acid sequence of amino acid numbers 28 to 380 of SEQ ID NO: 2.

The allergen domain used in the present invention includes Lit v 1, Lit v 4, and Lit v 3 as allergens. Lit v 1, Lit v 4, and Lit v 3 are allergens that are widely observed in crustaceans (Non-Patent Document 1). Lit v 1, Lit v 4, and Lit v 3 used in the present invention may be variants thereof as long as they have antigenicity. The antigenicity of any protein can be confirmed, for example, by observing that administration to an animal elicits antibody production or T cell response to that protein (Bioanalysis., 2012, Vol. 4, p. 397-406).

In one embodiment, Lit v 1 consists of the following amino acid sequence of (a) or (b):
(a) an amino acid sequence having at least 90% identity to the amino acid sequence of amino acid numbers 383 to 666 of SEQ ID NO: 2; or
(b) the amino acid sequence of amino acid numbers 383 to 666 of SEQ ID NO: 2, or an amino acid sequence in which 1 to 10 amino acids are deleted, substituted, inserted and/or added in the amino acid sequence of amino acid numbers 383 to 666 of SEQ ID NO: 2.

In one embodiment, Lit v 1 consists of the amino acid sequence of amino acid numbers 383 to 666 of SEQ ID NO: 2.

In one embodiment, Lit v 4 consists of the following amino acid sequence of (a) or (b):
(a) an amino acid sequence having at least 90% identity to the amino acid sequence of amino acid numbers 671 to 863 of SEQ ID NO: 2; or
(b) the amino acid sequence of amino acid numbers 671 to 863 of SEQ ID NO: 2, or an amino acid sequence in which 1 to 10 amino acids are deleted, substituted, inserted and/or added in the amino acid sequence of amino acid numbers 671 to 863 of SEQ ID NO: 2.

In one embodiment, Lit v 4 consists of the amino acid sequence of amino acid numbers 671 to 863 of SEQ ID NO: 2.

In one embodiment, Lit v 3 consists of the following amino acid sequence of (a) or (b):
(a) an amino acid sequence having at least 90% identity to the amino acid sequence of amino acid numbers 868 to 1044 of SEQ ID NO: 2; or
(b) the amino acid sequence of amino acid numbers 868 to 1044 of SEQ ID NO: 2, or an amino acid sequence in which 1 to 10 amino acids are deleted, substituted, inserted and/or added in the amino acid sequence of amino acid numbers 868 to 1044 of SEQ ID NO: 2.

In one embodiment, Lit v 3 consists of the amino acid sequence of amino acid numbers 868 to 1044 of SEQ ID NO: 2.

In one embodiment, the allergen domain used in the present invention includes Lit v1, Lit v 4 and Lit v 3 in any order. Also, in one embodiment, the allergen domain used in the present invention includes Lit v1, Lit v 4 and Lit v 3 in this order.

In one embodiment, the allergen domain used in the present invention consists of the amino acid sequence of amino acid numbers 383 to 1044 of SEQ ID NO: 2.

The general structure of the transmembrane domain is well known to those skilled in the art (Annu Rev Biochem., 2007, Vol. 76, p. 125 to 140). The transmembrane domain has a function of anchoring proteins to biological membranes. As the transmembrane domain used in the present invention, any suitable transmembrane domain protein can be selected as long as it has a function of anchoring proteins to biological membranes. In one embodiment, the transmembrane domain used in the present invention is a transmembrane domain of LAMP. In one embodiment, the transmembrane domain of LAMP used in the present invention is a transmembrane domain of LAMP-1.

In one embodiment, the transmembrane domain used in the present invention consists of the following amino acid sequence of (a) or (b):
(a) an amino acid sequence having at least 90% identity to the amino acid sequence of amino acid numbers 1048 to 1070 of SEQ ID NO: 2; or
(b) the amino acid sequence of amino acid numbers 1048 to 1070 of SEQ ID NO: 2, or an amino acid sequence in which 1 to 2 amino acids are deleted, substituted, inserted and/or added in the amino acid sequence of amino acid numbers 1048 to 1070 of SEQ ID NO: 2.

In one embodiment, the transmembrane domain used in the present invention consists of the amino acid sequence of amino acid numbers 1048 to 1070 of SEQ ID NO: 2.

The structure of the endosomal/lysosomal targeting domain of LAMP is well known to those skilled in the art (WO 1994/017192). The endosomal/lysosomal targeting domain of LAMP has a function of transporting a protein to lysosome. As the endosomal/lysosomal targeting domain of LAMP used in the present invention, any suitable endosomal/lysosomal targeting domain of LAMP can be selected as long as it has a function of transporting the protein to lysosome. In one embodiment, endosomal/lysosomal targeting domain of LAMP used in the present invention is an endosomal/lysosomal targeting domain of LAMP-1.

In one embodiment, the endosomal/lysosomal targeting domain of LAMP used in the present invention consists of the amino acid sequence of amino acid numbers 1079 to 1082 of SEQ ID NO: 2, or an amino acid sequence in which 1 amino acid is deleted, substituted, inserted and/or added in the amino acid sequence of amino acid numbers 1079 to 1082 of SEQ ID NO: 2.

In one embodiment, the endosomal/lysosomal targeting domain of LAMP used in the present invention consists of an amino acid sequence in a range of amino acid numbers 1079 to 1082 of SEQ ID NO: 2.

In the chimeric protein relating to the present invention, the signal peptide, the intra-organelle stabilizing domain of LAMP, each allergen comprised in the allergen domain, the transmembrane domain, and the endosomal/lysosomal targeting domain of LAMP may be directly linked or may be indirectly linked via a linker peptide. The linker peptide to be used can be appropriately selected by those skilled in the art. In one embodiment, the linker peptide consists of 10 or less amino acids. In one embodiment, a linker peptide used between the intra-organelle stabilizing domain of LAMP and the allergen domain, between allergens, and between the allergen domain and the transmembrane domain is a linker peptide selected from the group consisting of LeuGlu, GlyGlyGlyGly, and GluPheThr. In one embodiment, the linker peptide used between the transmembrane domain and the endosomal/lysosomal targeting domain of LAMP is a linker peptide consisting of the amino acid sequence of amino acid numbers 1071 to 1078 of SEQ ID NO: 2.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide of LAMP,
a nucleotide sequence encoding an intra-organelle stabilizing domain of LAMP;
a nucleotide sequence encoding an allergen domain comprising Lit v 1, Lit v 4, and Lit v 3;
a nucleotide sequence encoding a transmembrane domain of LAMP, and
a nucleotide sequence encoding an endosomal/lysosomal targeting domain of LAMP.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide of LAMP-1,
a nucleotide sequence encoding the intra-organelle stabilizing domain of LAMP-1,
a nucleotide sequence encoding an allergen domain comprising Lit v 1, Lit v 4, and Lit v 3;
a nucleotide sequence encoding a transmembrane domain of LAMP-1, and
a nucleotide sequence encoding an endosomal/lysosomal targeting domain of the LAMP-1.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2,
a nucleotide sequence encoding a peptide linker consisting of the amino acid sequence of amino acid numbers 1071 to 1078 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide;
a nucleotide sequence encoding an intra-organelle stabilizing domain of LAMP;
a nucleotide sequence encoding an allergen domain comprising Lit v 1, Lit v 4, and Lit v 3 in this order,
a nucleotide sequence encoding a transmembrane domain; and
a nucleotide sequence encoding an endosomal/lysosomal targeting domain of LAMP.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide of LAMP,
a nucleotide sequence encoding an intra-organelle stabilizing domain of LAMP;
a nucleotide sequence encoding an allergen domain comprising Lit v 1, Lit v 4, and Lit v 3 in this order,
a nucleotide sequence encoding a transmembrane domain of LAMP, and
a nucleotide sequence encoding an endosomal/lysosomal targeting domain of LAMP.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide of LAMP-1,
a nucleotide sequence encoding the intra-organelle stabilizing domain of LAMP-1,
a nucleotide sequence encoding an allergen domain comprising Lit v 1, Lit v 4, and Lit v 3 in this order,
a nucleotide sequence encoding a transmembrane domain of LAMP-1, and
a nucleotide sequence encoding an endosomal/lysosomal targeting domain of the LAMP-1.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2, in this order;
a nucleotide sequence encoding a transmembrane domain consisting of the amino acid sequence of amino acid numbers 1048 to 1070 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the nucleic acid of the present invention is the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2, in this order;
a nucleotide sequence encoding a transmembrane domain consisting of the amino acid sequence of amino acid numbers 1048 to 1070 of SEQ ID NO: 2,
a nucleotide sequence encoding a peptide linker consisting of the amino acid sequence of amino acid numbers 1071 to 1078 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

The nucleic acid of the present invention is not particularly limited as long as it encodes the chimeric protein relating to the present invention, and has an action of inducing Th1-type immunity with respect to the allergen selected from the group consisting of Lit v 1, Lit v 4, and Lit v 3, when the nucleic acid is administered to a human or an animal. One can confirm whether or not a certain nucleic acid has an action of inducing Th1-type immunity with respect to the allergen described above, when the nucleic acid is administered to a human or an animal, by the method described in, for example, Example 3 and/or Example 4. In addition, the nucleic acid of the present invention may be a nucleic acid having an action of inducing Th1 cell dominant immune response, when the nucleic acid is administered to a human or an animal. One can confirm whether or not a certain nucleic acid has an action of inducing Th1 cell dominant immune response, when the nucleic acid is administered to human or animal, by the method described in, for example, Example 4.

In one embodiment, the nucleic acid of the present invention is the following nucleic acid:
a nucleic acid comprising a nucleotide sequence encoding a chimeric protein consisting of an amino acid sequence having at least 90%, 92%, 94%, 96%, 98%, or 99% identity to the amino acid sequence shown by SEQ ID NO: 2.

In one embodiment, the nucleic acid of the present invention is the following nucleic acid:
a nucleic acid comprising a nucleotide sequence encoding a chimeric protein consisting of an amino acid sequence having at least 90% identity to the amino acid sequence shown by SEQ ID NO: 2.

In one embodiment, the nucleic acid of the present invention is the following nucleic acid:
a nucleic acid comprising a nucleotide sequence encoding a chimeric protein consisting of the amino acid sequence shown by SEQ ID NO: 2, or a chimeric protein consisting of an amino acid sequence in which 1 to 10 amino acids are deleted, substituted, inserted, and/or added in the amino acid sequence shown by SEQ ID NO: 2.

In one embodiment, the nucleic acid of the present invention is the following nucleic acid:
a nucleic acid comprising a nucleotide sequence encoding a chimeric protein consisting of an amino acid sequence having at least 90% identity to an amino acid sequence shown by SEQ ID NO: 2,
wherein the nucleic acid has an action of inducing Th1-type immunity to the allergen selected from the group consisting of Lit v 1, Lit v 4, and Lit v 3.

In one embodiment, the nucleic acid of the present invention is the following nucleic acid:
a) a nucleic acid comprising a nucleotide sequence encoding a chimeric protein consisting of an amino acid sequence shown by SEQ ID NO: 2; or
b) a nucleic acid comprising a nucleotide sequence encoding a chimeric protein consisting of an amino acid sequence in which 1 to 10 amino acids are deleted, substituted, inserted, and/or added in the amino acid sequence shown by SEQ ID NO: 2, wherein the nucleic acid has an action of inducing Th1-type immunity to an allergen selected from the group consisting of Lit v 1, Lit v 4, and Lit v 3.

In one embodiment, the nucleic acid of the present invention is the following nucleic acid:
a nucleic acid comprising a nucleotide sequence encoding a chimeric protein consisting of an amino acid sequence having at least 90% identity to an amino acid sequence shown by SEQ ID NO: 2, wherein the nucleic acid has an action of inducing Th1-type immunity to Lit v 1, Lit v 4, and Lit v 3.

In one embodiment, the nucleic acid of the present invention is the following nucleic acid:
a) a nucleic acid comprising a nucleotide sequence encoding a chimeric protein consisting of an amino acid sequence shown by SEQ ID NO: 2; or
b) a nucleic acid comprising a nucleotide sequence encoding a chimeric protein consisting of an amino acid sequence in which 1 to 10 amino acids are deleted, substituted, inserted, and/or added in the amino acid sequence shown by SEQ ID NO: 2, wherein the nucleic acid has an action of inducing Th1-type immunity to Lit v 1, Lit v 4, and Lit v 3.

In one embodiment, the nucleic acid of the present invention is the following nucleic acid:
a nucleic acid comprising a nucleotide sequence encoding a chimeric protein consisting of an amino acid sequence shown by SEQ ID NO: 2.

In one embodiment, the nucleotide sequence encoding the chimeric protein consisting of the amino acid sequence shown by SEQ ID NO: 2 means the nucleotide sequence shown by SEQ ID NO: 1.

Based on the nucleotide sequence, the nucleic acid of the present invention can be easily prepared by those skilled in the art by using methods known in the art. For example, the nucleic acid of the present invention can be synthesized by using gene synthesis methods known in the art. As such a gene synthesis method, various methods known to those skilled in the art such as a method for synthesizing an antibody gene described in WO 90/07861 can be used.

Once being synthesized, the nucleic acid of the present invention can be easily replicated by those skilled in the art using methods known in the art. For example, the nucleic acid of the present invention can be replicated by the method described later in <Method for producing the nucleic acid of the present invention and nucleic acid which can be produced by the method>.

### <Expression vector of the present invention>

The expression vector of the present invention includes an expression vector comprising the nucleic acid of the present invention.

The expression vector used to express a chimeric protein from the nucleic acid of the present invention is not particularly limited as long as it can express the chimeric protein from the nucleic acid of the present invention in the animal cells. In one embodiment, the expression vector used to express a chimeric protein from the nucleic acid of the present invention is an expression vector which can be used for expressing the chimeric protein in a human body. Examples of the expression vector used in the present invention include a plasmid vector, a viral vector (for example, adenovirus, retrovirus, adeno-associated virus) and the like. In one embodiment, the expression vector of the present invention is a plasmid vector. In the present specification, "plasmid" means the plasmid vector.

The expression vector of the present invention may comprise a promoter operably linked to the nucleic acid of the present invention. Examples of the promoter for expressing the chimeric protein from the nucleic acid of the present invention in animal cells include a virus-derived promoter such as CMV (cytomegalovirus), RSV (respiratory syncytial virus), and SV40 (simian virus 40), an actin promoter, EF (elongation factor) 1α promoter, a heat shock promoter and the like. In one embodiment, the promoter comprised in the expression vector of the present invention is a CMV promoter. The expression vector of the present invention may comprise a start codon and a stop codon. In this case, an enhancer sequence, an untranslated region, a splicing junction, a polyadenylation site, or a replicable unit or the like may be comprised.

In one embodiment, the expression vector of the present invention is an expression vector comprising the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the expression vector of the present invention is an expression vector comprising the following nucleic acid:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2,
a nucleotide sequence encoding a peptide linker consisting of the amino acid sequence of amino acid numbers 1071 to 1078 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the expression vector of the present invention is an expression vector comprising a nucleic acid comprising a nucleotide sequence encoding a chimeric protein consisting of the amino acid sequence shown by SEQ ID NO: 2.

In one embodiment, the expression vector of the present invention is an expression vector comprising a nucleic acid comprising the nucleotide sequence shown by SEQ ID NO: 1.

In one embodiment, the expression vector of the present invention is an expression vector comprising a nucleic acid consisting of the nucleotide sequence shown by SEQ ID NO: 3.

### <Host cell of the present invention>

The host cell of the present invention includes a host cell transformed with the nucleic acid of the present invention. In one embodiment, the host cell of the present invention is a host cell transformed with the expression vector of the present invention. In one embodiment, the host cell of the present invention is a host cell transformed with the expression vector of the present invention which is a plasmid vector.

The host cell transformed with the nucleic acid of the present invention is not particularly limited, and any cell known in the art can be selected as long as it is a cell that can be used for nucleic acid replication.

Examples of the host cell that can be used for nucleic acid replication include various cells such as natural cells or artificially established cells commonly used in the technical field of the present invention (for example, animal cells (for example, CHOK1SV cells and the like), insect cells (for example, Sf9 and the like), bacteria (for example, E. coli and the like), and yeasts (for example, Saccharomyces, Pichia, and the like), and the like). In one embodiment, E. coli can be used as a host cell. Transformation itself can be carried out by known methods.

### <Method for producing the nucleic acid of the present invention and nucleic acid which can be produced by the method>

Examples of the method for producing the nucleic acid of the present invention include a method for producing a nucleic acid or an expression vector, which comprises a step of culturing host cells transformed with the nucleic acid or the expression vector of the present invention. In one embodiment, the method for producing the nucleic acid of the present invention comprises a step of culturing the host cell transformed with the nucleic acid of the present invention, and replicating the nucleic acid of the present invention.

In one embodiment, the method for producing the nucleic acid of the present invention comprises a step of culturing the host cell transformed with the expression vector of the present invention, and replicating the expression vector of the present invention.

In one embodiment, the host cell used in the method for producing the nucleic acid of the present invention is E. coli. For culture of E. coli, an appropriate culture medium such as LB medium, M9 medium, Terrific Broth medium, SOB medium, SOC medium, or 2 × YT medium can be selected. In addition, the culturing of E. coli can be carried out in an environment where carbon (it is not particularly limited as long as it is an assimilable carbon compound; for example, polyols such as glycerin, or organic acids such as pyruvic acid, succinic acid, or citric acid), nitrogen (it is not particularly limited as long as it is a nitrogen compound that can be used by E. coli; for example, peptone, meat extract, yeast extract, casein hydrolysate, soybean meal alkaline extract, or ammonia or a salt thereof), inorganics and inorganic ions (it is not particularly limited, and examples thereof include phosphate, carbonate, sulfate, magnesium, calcium, potassium, iron, manganese and zinc), a vitamin source, an antifoaming agent, and the like are controlled to an appropriate concentration. In addition, the control of culturing includes control of parameters such as pH, temperature, stir, air flow and dissolved oxygen. In one embodiment, the conditions of culturing include pH of 6.7 to 7.5, temperature of 20°C to 37°C, and a stirring speed of 200 to 300 rpm.

The method for producing the nucleic acid of the present invention may comprise a step of obtaining lysate from collected culture solutions. The lysate can be obtained, for example, by treating the collected culture solutions with an alkaline lysis method or boiling method. Also, the step of obtaining the lysate may include a step of sterile filtration of a final lysate material.

The method for producing the nucleic acid of the present invention may further comprise a step of purifying nucleic acid or an expression vector from lysate. Ion exchange chromatography and/or hydrophobic interaction chromatography can be used to purify the nucleic acid or the expression vector from the lysate. The step of purifying the nucleic acid or the expression vector from the lysate may include a step of ultrafiltration and/or diafiltration. In addition, as a final treatment of the purification step, a sterile filtration step may be comprised.

In one embodiment, the nucleic acid of the present invention is a nucleic acid produced by the method for producing the nucleic acid of the present invention.

In one embodiment, the expression vector of the present invention is an expression vector produced by the method for producing the nucleic acid of the present invention.

### <Pharmaceutical composition of the present invention>

The pharmaceutical composition of the present invention includes a pharmaceutical composition comprising the nucleic acid of the present invention and a pharmaceutically acceptable excipient. In one embodiment, the pharmaceutical composition of the present invention is a pharmaceutical composition comprising the vector of the present invention and the pharmaceutically acceptable excipient. The pharmaceutical composition of the present invention can be prepared by a generally used method with an excipient generally used in the field, that is, a pharmaceutical excipient, a pharmaceutical carrier or the like. Examples of dosage forms of these pharmaceutical compositions include, for example, parenteral agents such as injections and drip agents, which can be administered by intravenous administration, subcutaneous administration, intradermal administration, intramuscular administration, and the like. In formulating, excipients, carriers, additives, and the like can be used according to these dosage forms within the pharmaceutically acceptable range.

In one embodiment, the pharmaceutical composition of the present invention is a pharmaceutical composition comprising the nucleic acid or the expression vector of the present invention and the pharmaceutically acceptable excipient.

While the administration amount of the nucleic acid of the present invention or the expression vector varies depending on the degree of symptoms and age of the patient, the dosage form of the preparation used and the like, for example, the amount in a range of 0.001 mg/kg to 100 mg/kg can be used. Further, it is possible to prepare a formulation by adding the nucleic acid or the expression vector of the present invention in an amount corresponding to such administration amount.

The pharmaceutical composition of the present invention can be used as an agent for preventing or treating allergy caused by an allergen selected from Lit v 1, Lit v 4 and Lit v 3. The pharmaceutical composition of the present invention can also be used as an agent for preventing or treating the crustacean allergy.

The present invention includes a pharmaceutical composition for preventing or treating allergy, comprising the nucleic acid of the present invention. The present invention also includes a method for preventing or treating allergy, comprising administering a prophylactically effective or therapeutically effective amount of the nucleic acid of the present invention. The present invention also includes the nucleic acid of the present invention for use in preventing or treating allergy. In addition, the present invention includes use of the nucleic acid of the present invention for manufacturing a pharmaceutical composition for preventing or treating allergy. In one embodiment, the above-described allergy is allergy caused by an allergen selected from the group consisting of Lit v 1, Lit v 4 and Lit v 3. In addition, in one embodiment, the above-described allergy is allergy affecting an allergy patient having an antibody that responds to an allergen selected from the group consisting of Lit v 1, Lit v 4, and Lit v 3. Further, in one embodiment, the above-described allergy is crustacean allergy.

In one embodiment, the pharmaceutical composition of the present invention is a pharmaceutical composition for preventing or treating allergy, comprising the following nucleic acid and a pharmaceutically acceptable excipient:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the pharmaceutical composition of the present invention is a pharmaceutical composition for preventing or treating allergy, comprising a nucleic acid comprising a nucleotide sequence encoding a chimeric protein consisting of the amino acid sequence shown by SEQ ID NO: 2 and a pharmaceutically acceptable excipient.

The present invention includes a pharmaceutical composition for preventing or treating allergy, comprising the expression vector of the present invention. In addition, the present invention includes a method for preventing or treating allergy, comprising administering a prophylactically effective or therapeutically effective amount of the expression vector of the present invention. The present invention also includes the expression vector of the present invention for use in preventing or treating allergy. In addition, the present invention includes use of the expression vector of the present invention for manufacturing a pharmaceutical composition for preventing or treating allergy. In one embodiment, the above-described allergy is allergy caused by an allergen selected from the group consisting of Lit v 1, Lit v 4 and Lit v 3. In addition, in one embodiment, the above-described allergy is allergy affecting an allergy patient having an antibody that responds to an allergen selected from the group consisting of Lit v 1, Lit v 4, and Lit v 3. Further, in one embodiment, the above-described allergy is crustacean allergy.

In one embodiment, the pharmaceutical composition of the present invention is a pharmaceutical composition for preventing or treating allergy, comprising an expression vector comprising the following nucleic acid and a pharmaceutically acceptable excipient:
a nucleic acid comprising a nucleotide sequence encoding a chimeric protein, wherein the nucleotide sequence is a nucleotide sequence comprising the following nucleotide sequences in this order:
a nucleotide sequence encoding a signal peptide consisting of the amino acid sequence of amino acid numbers 1 to 27 of SEQ ID NO: 2,
a nucleotide sequence encoding an intra-organelle stabilizing domain of LAMP consisting of the amino acid sequence of amino acid numbers 28 to 380 of SEQ ID NO: 2,
a nucleotide sequence encoding an allergen domain comprising Lit v 1 consisting of the amino acid sequence of amino acid numbers 383 to 666 of SEQ ID NO: 2, Lit v 4 consisting of the amino acid sequence of amino acid numbers 671 to 863 of SEQ ID NO: 2, and Lit v 3 consisting of the amino acid sequence of amino acid numbers 868 to 1044 of SEQ ID NO: 2,
a nucleotide sequence encoding a transmembrane domain consisting of the amino acid sequence of amino acid numbers 1048 to 1070 of SEQ ID NO: 2, and
a nucleotide sequence encoding an endosomal/lysosomal targeting domain of LAMP consisting of the amino acid sequence of amino acid numbers 1079 to 1082 of SEQ ID NO: 2.

In one embodiment, the pharmaceutical composition of the present invention is a pharmaceutical composition for preventing or treating allergy, comprising an expression vector comprising a nucleic acid comprising a nucleotide sequence encoding a chimeric protein consisting of the amino acid sequence shown by SEQ ID NO: 2 and a pharmaceutically acceptable excipient.

Specific examples are provided herein for reference in order to obtain further understanding of the present invention; however, these examples are for the purpose of illustration and the present invention is not limited thereto.

### [Examples]

Unless otherwise specified, the steps described in the following examples can be implemented according to known methods. Moreover, in a case of using a commercially available reagent, kit, or the like, the above steps can be implemented according to the instruction manual of a commercially available product.

### [Example 1: Construction of LAMP-Lit v 1-Lit v 4-Lit v 3 plasmid]

LAMP-Lit v 1-Lit v 4-Lit v 3 plasmid consisting of the nucleotide sequence shown by SEQ ID NO: 3 (an expression vector comprising a nucleic acid comprising a nucleotide sequence comprising the following nucleotide sequences in this order (that is, a nucleotide sequence encoding a chimeric protein consisting of the amino acid sequence shown by SEQ ID NO: 2): a nucleotide sequence encoding a signal peptide of LAMP-1, a nucleotide sequence encoding an intra-organelle stabilizing domain of LAMP-1, a nucleotide sequence encoding an allergen domain comprising Lit v 1, Lit v 4, and Lit v 3 in this order, a nucleotide sequence encoding a transmembrane domain of LAMP-1, and a nucleotide sequence encoding an endosomal/lysosomal targeting domain of LAMP-1) was constructed. The plasmid can be constructed by inserting synthetic DNA, in which Xho I recognition sequence is added to 5' end of the nucleotide sequence of 1147 to 3132 of SEQ ID NO: 1 (a nucleotide sequence encoding an allergen domain comprising Lit v 1, Lit v 4, and Lit v 3 in this order) and Eco RI recognition sequence is added to the 3' end of the nucleotide sequence, into Eco RI-Xho I site of the plasmid shown by SEQ ID NO: 6 of Japanese Patent No. 5807994. E. coli was transformed with the constructed LAMP-Lit v 1-Lit v 4-Lit v 3 plasmid, and cultured in a liquid medium. The amplified LAMP-Lit v 1-Lit v 4-Lit v 3 plasmid was obtained by a method of centrifuging the culture solution and collecting the cells based on a general plasmid extraction and purification method (miniprep method).

### [Example 2: Expression of LAMP-Lit v 1-Lit v 4-Lit v 3 chimeric protein]

In vitro expression of the LAMP-Lit v 1-Lit v 4-Lit v 3 chimeric protein (a chimeric protein consisting of an amino acid sequence encoded by the nucleotide sequence shown by SEQ ID NO: 1 (that is, the amino acid sequence shown by SEQ ID NO: 2)) by using human embryonic kidney-derived 293T cell line was evaluated.

### (1) Cell culture and plasmid introduction

Human embryonic kidney-derived 293T cells (Thermo Fisher Scientific, Cat. HCL4517) were suspended in D-MEM medium (Sigma-Aldrich, Cat. D5796) containing 10% fetal bovine serum (Hyclone, Cat. SH30070.03) and 100-fold diluted penicillin-streptomycin (Thermo Fisher Scientific, Cat. 15070063), and cultured in 6-well plates (Cat. 3810-006 manufactured by IWAKI) at 3 × 10⁵ cells/well. After overnight culture at 37°C in the presence of 5% CO2, 250 µL of a mixed solution having a ratio of LAMP-Lit v 1-Lit v 4-Lit v 3 plasmid: Lipofectamine 2000 (Thermo Fisher Scientific, Cat. 11668027) = 2.5 (µg):10 (µL) was added. Further, after overnight culture at 37°C in the presence of 5% CO2 again, the medium was removed and washed once with PBS, and then western blotting was performed.

### (2) Western blotting

Sample preparation: Cells were lysed with a RIPA buffer (Pierce Cat. 89900) containing a protease inhibitor (Roche Diagnostics Cat. 11873580), centrifuged at 20,000 xg for 5 minutes, and after centrifugation, a protein concentration of a supernatant was measured using a DC protein assay kit II (Bio-Rad Cat. 500-0112). To 5 µL of the supernatant diluted with PBS containing protease inhibitor so that the protein concentration would be 200 µg/mL, 5 µL of LDS sample buffer (Thermo Fisher Scientific, Cat. NP0007) containing 100 mM DTT was added, and heat-treated at 70°C for 10 minutes so as to prepare a sample to be subjected to SDS-PAGE.

SDS-PAGE: the above-described samples were applied to NuPAGE (Registered trademark) 4%-12% Bis-Tris Gel (Thermo Fisher Scientific, Cat. NP0323) and electrophoresis was performed at a constant voltage of 200 V in NuPAGE (Registered trademark) MOPS SDS Running buffer (Thermo Fisher Scientific, Cat. NP0001). Blotting: Blotting was performed by bringing PVDF membrane (Thermo Fisher Scientific, Cat. LC2005) into contact with the gel after SDS-PAGE, and electrophoretic transferring for 70 minutes at 180 mA in XCell II Blot Module (Thermo Fisher Scientific, Cat. EI9051) filled with NuPAGE (Registered trademark) Transfer buffer (Thermo Fisher Scientific, Cat. NP0006) containing 20% of methanol.
Blocking: The membrane after electrophoresis was blocked with Blocking One (Nacalai Tesque, Cat. 03953-95) for one hour at room temperature.
Primary antibody: The membrane was incubated with a solution of Anti-human LAMP-1 antibody (Sino biological, Cat. 11215-RP01) diluted 1000-fold with TBS Tween-20 buffer (Thermo Fisher Scientific, Cat. 28360) containing 10% of Blocking One, and shaken overnight at 4°C.
Secondary antibody: The membrane was washed with TBS Tween-20 buffer. The membrane was incubated with a solution of Anti-rabbit IgG (H + L chain) pAb-HRP (MBL, Cat. 458) diluted 3000-fold with TBS Tween-20 buffer containing 10% of Blocking One and shaken at room temperature for one hour.
Detection: The membrane was washed with TBS Tween-20 buffer. The immunoblots was developed by ECL prime western blotting detection reagent (GE Healthcare, Cat. RPN2232), and an image was detected with LumiVision PRO 400EX (Aisin Seiki Co., Ltd.). In the image, a band responsive to the anti-human LAMP-1 antibody corresponding to the chimeric protein was detected.

As a result of the above test, by introducing the LAMP-Lit v 1-Lit v 4-Lit v 3 plasmid to the human embryonic kidney-derived 293T cell line, it was checked that LAMP-Lit v 1-Lit v 4-Lit v 3 chimeric protein was expressed in the cells.

### [Example 3: Induction of IgG2a antibody production by administration of LAMP-Lit v 1-Lit v 4-Lit v 3 plasmid]

In vivo evaluation of induction of IgG2a antibody production was performed in mice. In eight examples in each group, 25 µL of a PBS solution containing 5 µg of LAMP-Lit v 1-Lit v 4-Lit v 3 plasmid, as a multivalent plasmid group, was administered in the ear of 6-week-old BALB/c female mouse, which is at the start of administration (Charles River Laboratories Japan, Inc.), intradermally three times every week (Day 0, 7, and 14). Three weeks after the last dose (Day 35), blood was collected, and plasma samples were obtained. For comparison, as a monovalent plasmid mix group, 25 µL of PBS solution containing a mixtureof 5 µg of LAMP-Lit v 1 plasmid (an expression vector comprising a nucleic acid comprising the nucleotide sequence including the following nucleotide sequences in this order: a nucleotide sequence encoding an amino acid sequence of amino acid numbers 1 to 380 of SEQ ID NO: 2 (hereinafter, referred to as an N-terminal of LAMP-1 in Examples 3 and 4), a nucleotide sequence encoding a Lit v 1 allergen domain consisting of amino acid numbers 383 to 666 of SEQ ID NO: 2, and a nucleotide sequence encoding an amino acid sequence of amino acid numbers 1048 to 1082 of SEQ ID NO: 2 (hereinafter, referred to as a C-terminal of LAMP-1)), LAMP-Lit v 3 plasmid (an expression vector comprising a nucleic acid comprising the nucleotide sequence including the following nucleotide sequences in this order: a nucleotide sequence encoding an N-terminal of LAMP-1, a nucleotide sequence encoding a Lit v 3 allergen domain consisting of the amino acid sequence of amino acid numbers 868 to 1044 of SEQ ID NO: 2, and a nucleotide sequence encoding a C-terminal of LAMP-1), and LAMP-Lit v 4 plasmid (an expression vector comprising a nucleic acid comprising the nucleotide sequence including the following nucleotide sequences in this order: a nucleotide sequence encoding an N-terminal of LAMP-1, a nucleotide sequence encoding a Lit v 4 allergen domain consisting of the amino acid sequence of amino acid numbers 671 to 863 of SEQ ID NO: 2, and a nucleotide sequence encoding a C-terminal of LAMP-1) (hereinafter, referred to as a monovalent plasmid), and 25 µL of PBS as a control group were administered. An antibody titer was measured by an ELISA method using a plasma sample diluted 10-fold, 100-fold or 1000-fold with PBS containing 1% BSA (Sigma-Aldrich, Cat. A8022). The antibody titer was calculated by using standard plasma which contains a high amount of Lit v 1, Lit v 3, and Lit v 4 specific antibodies prepared from a mouse immunized with a shrimp antigen (Greer, Cat. RMF34P), and said standard plasma was serially diluted to prepare a calibration curve. At that time, the IgG2a antibody titer specific to each allergen in the standard plasma was set to 100 U/mL. ELISA measurement was performed based on a general ELISA method using F96 MAXISORP NUNC-IMMUNO PLATE (Nunc, Cat. 439454) as a test plate. Lit v 1 which is a natural shrimp extracted and purified protein (INDOOR Biotechnologies, Cat. NA-STM-1) was prepared at 1 µg/mL with PBS, and recombinant purified protein Lit v 4 (Sysmex, Uniprot KB: C7A639) and Lit v3 (Sysmex, Uniprot KB:B7SNI3) were prepared at 2 µg/mL with PBS, then added to the test plate at 50 µL/well, and incubated at 4°C overnight. After washing a test plate three times with a washing buffer (PBS Tween-20 buffer, Thermo Fisher Scientific, Cat. 28352), 100 µL/well of PBS containing 1% of BSA was added and incubated at room temperature for one hour. After washing the test plate three times with the washing buffer, the plasma sample was added at 50 µL/well and incubated at room temperature for one hour. After washing the test plate three times with the washing buffer, 50 µL/well of a 50000-fold diluted secondary antibody anti-Mouse IgG2a Detection Antibody (Bethyl Laboratories, Cat. A90-107P) in PBS containing 1% of BSA was added, and the test plate was incubated at room temperature for one hour. After washing the test plate three times with the washing buffer, a substrate solution TMB Microwell Peroxidase Substrate System (Ceracare, Cat. 50-76-03) was added at 50 µL/well, Lit v 1 was incubated at room temperature for 10 minutes, Lit v 3 was incubated at room temperature for 30 minutes, and Lit v 4 was incubated at room temperature for 60 minutes, and then a reaction stop solution (2M H₂SO₄) was added at 50 µL/well so as to measure absorbance at 450 nm.

As a result of the above test, by administering LAMP-Lit v 1-Lit v 4-Lit v 3 plasmid (multivalent plasmid) to mice, the production of Lit v 1, Lit v 3, and Lit v 4-specific IgG2a antibody, which are similar to those of monovalent plasmid mix administration, was confirmed (Fig. 1). From the above results, it has been suggested that LAMP-Lit v 1-Lit v 4-Lit v 3 plasmid induces Th1 immune responses for all the allergens encoded with the plasmid and causes class switch of activated B cells to the IgG2a isotype.

### [Example 4: Induction of IFN-γ and IL-4 production by LAMP-Lit v 1-Lit v 4-Lit v 3 plasmid]

Induction of IFN-γ and IL-4 production upon stimulation with allergen from splenocytes collected from mice administered with LAMP-Lit v 1-Lit v 4-Lit v 3 plasmid was evaluated. The mice administered with the LAMP-Lit v 1-Lit v 4-Lit v 3 plasmid used in Example 3 were sacrificed 3 weeks after the final administration (Day 35), and the spleen was sampled. Splenocytes were prepared from the spleen according to a general method. The prepared splenocytes were suspended in RPMI-1640 medium (Sigma-Aldrich, Cat. R8758) containing 10% fetal bovine serum (Hyclone, Cat. SH30070.03) and 100-fold diluted penicillin-streptomycin (ThermoFisher, Cat. 15070063), and cultured in 96-well plates at 8 × 10⁵ cells/well. For the allergen stimulation, a shrimp extract solution (allergic scratch extract for diagnosis, Torii, approval number (40A) 4688) or Lit v 1 (INDOOR Biotechnologies, Cat. NA-STM-1) was used. The allergen stimulation was conducted by adding the shrimp extract solution or Lit v 1 into wells at a final concentration of 100 µg/mL or 10 µg/mL, respectively, and cultured at 37°C under 5% CO₂ for 72 hours. Then the concentration of IFN-γ and IL-4 released from the splenocytes into the culture supernatant upon stimulation was measured by the ELISA method. A supernatant sample diluted 10-fold with TBS buffer containing 0.1% BSA and 0.05% Tween 20 was used for the measurement of IFN-γ, and a supernatant sample diluted 3-fold with PBS buffer containing 1% BSA was used for the measurement of IL-4. As a test plate for ELISA measurement, F96 MAXISORP NUNC-IMMUNO PLATE was used. As an ELISA kit, mouse IFN-γ DuoSet ELISA (R&D Systems, Cat. DY485) and mouse IL-4 DuoSet ELISA (R&D Systems, Cat. DY 404) was used. The test was performed according to the protocol attached to the ELISA kit. In the above test, administration of LAMP-Lit v 1-Lit v 4-Lit v 3 plasmid (multivalent plasmid) induced shrimp extract solution or Lit v 1-specific IFN-γ production to the same extent as administration of monovalent plasmid mix administration (Fig. 2). In contrast, the amount of IL-4 induced in the shrimp extract solution or Lit v 1 by administering the multivalent plasmid and monovalent plasmid mix to the mouse was under the lower limit of detection (Fig. 3). From the above results, it was suggested that the LAMP-Lit v 1-Lit v 4-Lit v 3 plasmid induces a Th1 type immune response in which Th1 cells are dominant.

### [Example 5: Suppressive effect of shrimp antigen-induced anaphylactic symptom by LAMP-Lit v 1-Lit v 4-Lit v 3 plasmid]

In order to determine the therapeutic effect of the LAMP-Lit v 1-Lit v 4-Lit v 3 plasmid on allergy, the suppressive effect on anaphylactic symptoms was evaluated by the method described in detail below.

### (1) Transdermal sensitization of shrimp antigen

The hair on the back of a 7-week-old BALB/c female mouse (Japan Charles River) was shaved with a clipper, and 60 µL of 4% SDS (Invitrogen, Cat. 15553035) prepared with milliQ water was applied to the back of the anesthetized mouse by a pipette. After returning to a cage and air-drying, a shrimp antigen (GREER, Cat. RNF34P) prepared with 1.5% NaHCO₃/PBS to a protein concentration of 5 mg/mL was applied to the back of the anesthetized mouse by a pipette in an amount of 0.3 mg/60 µL. As a control, six examples of mice were coated with 60 µL of 1.5% NaHCO₃/PBS instead of the shrimp antigen. This series of operations was carried out three times a week for two weeks from the application start date (Day 0, 2, 4, 7, 9, and 11). In order to make the sensitization level of each group uniform, plasma samples were collected on Day 14, and Lit v 1-specific IgE, IgG1, and IgG2a antibody titers were measured according to the method described in Example 3 and the general measurement method described in a book (immunoassay, from basic to advanced, Biochemical Assay Research Group, Norihiro Kobayashi, 2014), and grouping was performed by the equal number method.

### (2) Intradermal immunity with LAMP-Lit v 1-Lit v 4-Lit v 3 plasmid

Into the ears of eight of shrimp antigen-sensitized mice grouped in (1), 0.5 µg, 5 µg, 50 µg of LAMP-Lit v 1-Lit v 4-Lit v 3 plasmid in 20 µL PBS or 20 µL of PBS as a control was intradermally administered four times per week (Day 21, 28, 35, and 42). Twenty µL of PBS was similarly intradermally administered to six examples of non-sensitized mice.

### (3) Induction of allergic symptoms by shrimp antigen

On Day 50, the prepared shrimp antigen at 0.5 mg/mL with 1.5% NaHCO₃/PBS was intraperitoneally administered at 0.1 mg/200 µL per mouse, rectal temperatures were measured before the administration, and 15 minutes, 30 minutes, 45 minutes, and 60 minutes after the administration of the antigen using a thermometer for mice (A&D Company, Cat. AD-1687). Moreover, the allergic symptom observed 60 minutes after the antigen administration was scored from 1 to 5. The judgement of the symptom was performed based on criteria written in the document (The Journal of Allergy and Clinical Immunology, 2013, Vol. 131, p. 213-221).

In order to measure a degree of mast cell degranulation, plasmas were collected 60 minutes after the induction of allergic symptoms, and the plasma concentration of mouse mast cell protease 1 (mMCPT-1) was measured. As the ELISA kit, mouse MCPT-1 Uncoated ELISA (Thermo Fisher Scientific, Cat. 88-7503) was used, and the measurement was performed in accordance with the attached protocol. As a test plate for ELISA measurement, F96 MAXISORP NUNC-IMMUNO PLATE was used. As measurement values, plasma samples diluted 10-fold, 200-fold, and 40,000-fold with a diluent contained in the kit were used for the measurement, and dilution results within a standard sample calibration curve range contained in the kit were selected.

From the results of the above test, it was shown that the LAMP-Lit v 1-Lit v 4-Lit v 3 plasmid suppressed the decrease in body temperature due to shrimp antigen administration and alleviated allergic symptoms compared with the control group in mouse (Fig. 4). In addition, it was confirmed that plasma degranulation 60 minutes after symptom induction was suppressed by administration of LAMP-Lit v 1-Lit v 4-Lit v 3 plasmid (Fig. 5). These results indicate that the LAMP-Lit v 1-Lit v 4-Lit v 3 plasmid can be used for a therapeutic treatment.

### Industrial Applicability

The nucleic acid of the present invention is expected to be useful for the preventing or treating of crustacean allergy. In addition, the method for producing the nucleic acid of the present invention is useful for producing the nucleic acid.

### Sequence Listing Free Text

The numerical heading <223> in the following sequence listing describes the description of "Artificial Sequence". Specifically, the nucleotide sequence shown by SEQ ID NO: 1 in the sequence listing is a nucleotide sequence encoding LAMP-Lit v 1-Lit v 4-Lit v 3 chimeric protein, and the amino acid sequence shown by SEQ ID NO: 2 in the sequence listing is the amino acid sequence encoded by SEQ ID NO: 1. The nucleotide sequence shown by SEQ ID NO: 3 is the nucleotide sequence of the LAMP-Lit v 1-Lit v 4-Lit v 3 plasmid.

## Claims

1. A nucleic acid comprising:
a nucleotide sequence encoding a chimeric protein,
wherein the nucleotide sequence is a nucleotide sequence comprising the following nucleotide sequences in this order:
a nucleotide sequence encoding a signal peptide;
a nucleotide sequence encoding an intra-organelle stabilizing domain of LAMP;
a nucleotide sequence encoding an allergen domain comprising Lit v 1, Lit v 4, and Lit v 3;
a nucleotide sequence encoding a transmembrane domain; and
a nucleotide sequence encoding an endosomal/lysosomal targeting domain of LAMP.

2. A nucleic acid comprising:
a nucleotide sequence encoding a chimeric protein,
wherein the nucleotide sequence is a nucleotide sequence comprising the following nucleotide sequences in this order:
a nucleotide sequence encoding a signal peptide;
a nucleotide sequence encoding an intra-organelle stabilizing domain of LAMP;
a nucleotide sequence encoding an allergen domain comprising Lit v 1, Lit v 4, and Lit v 3 in this order;
a nucleotide sequence encoding a transmembrane domain; and
a nucleotide sequence encoding an endosomal/lysosomal targeting domain of LAMP.

3. The nucleic acid according to claim 1 or 2, wherein the signal peptide is a signal peptide of LAMP.

4. The nucleic acid according to any one of claims 1 to 3, wherein the transmembrane domain is a transmembrane domain of LAMP.

5. The nucleic acid according to any one of claims 1 to 4, wherein the signal peptide consists of an amino acid sequence of amino acid numbers 1 to 27 of SEQ ID NO: 2, the intra-organelle stabilizing domain consists of an amino acid sequence of amino acid numbers 28 to 380 of SEQ ID NO: 2, the allergen domain is an allergen domain comprising Lit v 1 consisting of an amino acid sequence of amino acid numbers 383 to 666 of SEQ ID NO: 2, Lit v 4 consisting of an amino acid sequence of amino acid numbers 671 to 863 of SEQ ID NO: 2, and Lit v 3 consisting of an amino acid sequence of amino acid numbers 868 to 1044 of SEQ ID NO: 2, the transmembrane domain consists of an amino acid sequence of amino acid numbers 1048 to 1070 of SEQ ID NO: 2, and the endosomal/lysosomal targeting domain consists of an amino acid sequence of amino acid numbers 1079 to 1082 of SEQ ID NO: 2.

6. A nucleic acid comprising:
a nucleotide sequence encoding a chimeric protein consisting of an amino acid sequence having at least 90% identity to an amino acid sequence shown by SEQ ID NO: 2, wherein the nucleic acid has an action of inducing Th1-type immunity to an allergen selected from the group consisting of Lit v 1, Lit v 4, and Lit v 3.

7. A nucleic acid comprising:
a) a nucleotide sequence encoding a chimeric protein consisting of an amino acid sequence shown by SEQ ID NO: 2; or
b) a nucleotide sequence encoding a chimeric protein consisting of an amino acid sequence in which 1 to 10 amino acids are deleted, substituted, inserted, and/or added in the amino acid sequence shown by SEQ ID NO: 2, wherein the nucleic acid has an action of inducing Th1-type immunity to an allergen selected from the group consisting of Lit v 1, Lit v 4, and Lit v 3.

8. A nucleic acid comprising:
a nucleotide sequence encoding a chimeric protein consisting of an amino acid sequence shown by SEQ ID NO: 2.

9. An expression vector comprising:
the nucleic acid according to any one of claims 1 to 8.

10. An expression vector comprising:
the nucleic acid according to claim 8.

11. A host cell transformed with the nucleic acid according to any one of claims 1 to 8.

12. A method for producing a nucleic acid, comprising:
culturing a host cell transformed with the nucleic acid according to any one of claims 1 to 8.

13. A pharmaceutical composition comprising:
the expression vector according to claim 10 and a pharmaceutically acceptable excipient.

14. The pharmaceutical composition according to claim 13, which is a pharmaceutical composition for preventing or treating crustacean allergy.

15. A method for preventing or treating crustacean allergy, comprising:
administering a prophylactically effective or therapeutically effective amount of the expression vector according to claim 10.

16. The expression vector according to claim 10, for use in preventing or treating crustacean allergy.

17. Use of the expression vector according to claim 10 for the manufacture of a pharmaceutical composition for preventing or treating crustacean allergy.
